# EUROPEAN PATENT SPECIFICATION

(11) **EP 3 542 293 B1**
(45) Date of publication and mention of the grant of the patent: **27.12.2023**
(21) Application number: 17808277.2
(22) Date of filing: 15.11.2017
(51) Int. Cl.: G16B 30/00, G16B 30/10

(54) **METHODS OF SEQUENCING DATA READ REALIGNMENT**
VERFAHREN ZUR ABLESENEUAUSRICHTUNG VON SEQUENZIERUNGSDATEN
PROCÉDÉS DE RÉALIGNEMENT DE LECTURE DE DONNÉES DE SÉQUENÇAGE

(30) Priority: 16.11.2016 US 201662422841 P; 17.01.2017 US 201762447103 P; 31.03.2017 US 201762480330 P
(43) Date of publication of application: 25.09.2019
(60) Divisional application: 23207604.2
(73) Proprietor: Illumina, Inc., San Diego, CA 92122 (US)
(72) Inventor: BERRY, Gwenn, San Diego, California 92122 (US); CHUANG, Han-Yu, San Diego, California 92122 (US); GORDON, Jessica, San Diego, California 92122 (US); CHEN, Xiao, San Diego, California 92122 (US); TANNER, Stephen, San Diego, California 92122 (US)
(74) Representative: Patentwerk B.V.
(86) International application number: PCT/US2017/061661
(87) International publication number: WO 2018/093804

(56) References cited:
- US-A1- 2012 203 792
- ANDY RIMMER ET AL: "Integrating mapping-, assembly- and haplotype-based approaches for calling variants in clinical sequencing applications", NATURE GENETICS, vol. 46, no. 8, 1 August 2014 (2014-08-01) , pages 912-918, XP055158556, ISSN: 1061-4036, DOI: 10.1038/ng.3036
- C. A. ALBERS ET AL: "Dindel: Accurate indel calls from short-read data", GENOME RESEARCH, vol. 21, no. 6, 1 June 2011 (2011-06-01), pages 961-973, XP055206270, ISSN: 1088-9051, DOI: 10.1101/gr.112326.110
- P. KRAWITZ ET AL: "Microindel detection in short-read sequence data", BIOINFORMATICS, vol. 26, no. 6, 9 February 2010 (2010-02-09), pages 722-729, XP055186017, ISSN: 1367-4803, DOI: 10.1093/bioinformatics/btq027
- Frank Nothaft: "Scalable Genome Resequencing with ADAM and avocado", , 12 March 2015 (2015-03-12), XP055449063, Retrieved from the Internet: URL:http://digitalassets.lib.berkeley.edu/ techreports/ucb/text/EECS-2015-65.pdf
- MARK A DEPRISTO ET AL: "A framework for variation discovery and genotyping using next-generation DNA sequencing data", NATURE GENETICS, vol. 43, no. 5, 1 January 2011 (2011-01-01), pages 491-498, XP055046798, ISSN: 1061-4036, DOI: 10.1038/ng.806
- ANDY RIMMER ET AL: "Integrating mapping-, assembly- and haplotype-based approaches for calling variants in clinical sequencing applications", NATURE GENETICS., vol. 46, no. 8, 1 August 2014 (2014-08-01) , pages 912-918, XP055449632, NEW YORK, US ISSN: 1061-4036, DOI: 10.1038/ng.3036

## Description

### BACKGROUND

An ongoing challenge of next-generation sequencing data analysis is the accurate calling of insertions and deletions ("indels"). Reasons for this difficulty include a lower rate of occurrence, difficulty mapping to the correct location in the genome, and the presence of repeat regions in the genome which prevent unique mapping. Another reason is the inability or inaccuracy of current aligners to correctly identify variants at the ends of sequencing reads. This arises due to the lack of a two-sided context in which to place the variant call.

### SUMMARY

Shortcomings of the prior art are overcome and additional advantages are provided through the provision of a computer-implemented method, computer system, and computer program product. To offer an overview of the context of the present invention, reference is specifically made to the following relevant publications from the prior art: ANDY RIMMER ET AL: "Integrating mapping-, assembly- and haplotypebased approaches for calling variants in clinical sequencing applications", NATURE GENETICS, vol. 46, no. 8, 1 August 2014; C. A. ALBERS ET AL: "Dindel: Accurate indel calls from short-read data", GENOME RESEARCH, vol. 21, no. 6, 1 June 2011; and P. KRAWITZ ET AL: "Microindel detection in short-read sequence data", BIOINFORMATICS, vol. 26, no. 6, 9 February 2010.

According to a first embodiment of the invention as defined in appended claim 1, a computer-implemented method for sequencing data read realignment includes: obtaining from a sequence alignment dataset an initial alignment of a read sequence to a reference sequence, the initial alignment comprising an aligned read; performing realignment processing on the initial alignment, the realignment processing realigning the read sequence to the reference sequence to produce one or more candidate realignments, and the realignment processing comprising: identifying one or more candidate indels, the one or more candidate indels comprising zero or more indels in the aligned read and zero or more indels aligned proximal to the aligned read as indicated by the sequence alignment dataset; creating a flattened aligned read based at least on removing from the aligned read any indels indicated by the initial alignment; and determining one or more candidate realignments of the read sequence to the reference sequence based on introducing, for each candidate realignment of the one or more candidate realignments, a respective at least one candidate indel of the one or more candidate indels into the flattened aligned read; and providing the initial alignment or a selected candidate realignment of the one or more candidate realignments based on one or more selection criteria.

Further in regard of the first embodiment of the invention, the one or more candidate indels comprise a plurality of candidate indels, and the determining the one or more candidate realignments comprises commencing iteratively introducing the plurality of candidate indels into the flattened aligned read, wherein each iteration of the iteratively introducing provides a candidate realignment of the one or more candidate realignments by introducing into the flattened aligned read the respective at least one candidate indel for the candidate realignment.

The iteratively introducing can introduce permutations of one or more candidate indels of the plurality of candidate indels into the flattened aligned read to obtain, for each permutation of the permutations, a different candidate realignment of the one or more candidate alignments.

The realignment processing can further comprise: checking a provided candidate realignment of the one or more candidate realignments to determine whether and aligned read of the provided candidate realignment, the aligned read of the provided candidate realignment having the introduced respective one or more candidate indels, aligns with the reference sequence with no bases mismatched as between the aligned read of the provided candidate realignment and the reference sequence; halting the iteratively introducing based on determining that the aligned read of the provided candidate realignment aligns with the reference sequence with no bases mismatched; and selecting the provided candidate realignment as the selected candidate realignment, wherein the providing outputs the selected candidate realignment based on the aligned read of the provided candidate realignment aligning with the reference sequence.

The realignment processing can further comprise prioritizing the plurality of indels for the iteratively introducing, wherein the iteratively introducing introduces the plurality of indels in order of priority based on the prioritizing.

The prioritizing can prioritize an indel indicated by a reference indel dataset to be a prior known indel over an indel not indicated by the reference indel dataset to be a prior known indel. Additionally or alternatively, the prioritizing can prioritize an indel of longer length over an indel of shorter length. Additionally or alternatively, the prioritizing can prioritize an indel indicated in a greater number of aligned reads of the sequence alignment dataset over an indel indicated in a lesser number of aligned reads of the sequence alignment dataset. Additionally or alternatively, the prioritizing can prioritize an indel indicated in a greater proportion of aligned reads of the sequence alignment dataset that correspond to a location of the indel relative to the reference sequence over an indel indicated in a lesser proportion of aligned reads of the sequence alignment dataset. Additionally or alternatively, the prioritizing can prioritize, as between different indels indicated in a same number of aligned reads of the sequence alignment dataset, an indel whose location relative to a reference genomic sequence indicated by the sequence alignment dataset is upstream from a location, relative to the reference genomic sequence, indicated for another indel.

The selection criteria may be based at least in part on one or more of: number of bases mismatched, number of indels, location of indels relative to a reference genomic sequence indicated by the sequence alignment dataset, and number of softclipped bases.

The selection criteria can prioritize one or more of: an alignment having no indels and only a single base mismatched over an alignment having one or more indels for the providing; an alignment having a lesser number of bases mismatched over an alignment having a greater number of bases mismatched for the providing; as between different alignments having a same number of bases mismatched, an alignment having a lesser number of softclips of a specified type over an alignment having a greater number of softclips of the specified type for the providing; and as between different alignments having a same number of bases mismatched, an alignment having a lesser number of indels over an alignment having a greater number of indels for the providing.

The realignment processing can further comprise selecting a best candidate realignment of the one or more candidate realignments based on a first criteria of the one or more selection criteria, wherein the selected candidate realignment is the selected best candidate realignment, and wherein the outputting selects between the initial alignment and the best realignment candidate based on a second criteria of the one or more selection criteria.

An embodiment of a computer-implemented method can further comprise determining whether the obtained initial alignment is eligible for realignment, the determining being based at least in part on one or more of: identifying whether there are one or more bases mismatched as between the aligned read of the initial alignment and the reference sequence; identifying whether the aligned read comprises a softclip; identifying whether the initial alignment is not a secondary alignment; and identifying whether there are candidate indels around the aligned read in a region of bases of a reference genomic sequence of the sequence alignment dataset.

An embodiment of a computer-implemented method can further comprise determining whether the obtained initial alignment is eligible for realignment, and performing the realignment processing and the providing the initial alignment or selected candidate realignment based on determining that the obtained initial alignment is eligible for realignment; repeating, for each additional initial alignment of one or more additional initial alignments of the sequence alignment dataset, the obtaining and the determining whether the obtained additional initial alignment is eligible for realignment; and performing processing for each additional initial alignment of the one or more additional initial alignments, the performing processing comprising (i) providing the additional initial alignment as is, absent performing the realignment processing, or (ii) performing the realignment processing and the providing the additional initial alignment or selected candidate realignment.

Further, a computer system for sequencing data read realignment according to appended claim 11, comprising memory and at least one processor can be configured to execute program instructions to perform methods according to aspects described herein.

Yet further, a computer program product for sequencing data read realignment, comprising a tangible storage medium storing program instructions for execution can perform methods according to aspects described herein.

Additional features and advantages are realized through the concepts described herein. Numerous inventive aspects and features are disclosed herein, and unless inconsistent, each disclosed aspect or feature is combinable with any other disclosed aspect or feature as desired by a particular application, for instance, to facilitate detecting an image obstruction.

### BRIEF DESCRIPTION OF THE DRAWINGS

Aspects described herein are particularly pointed out and distinctly claimed as examples in the claims at the conclusion of the specification. The foregoing and other objects, features, and advantages of the invention are apparent from the following detailed description taken in conjunction with the accompanying drawings in which:
FIGS. 1A-1D illustrate how a two-sided context can be used to explain varying bases toward an end of a read;
FIG. 2 illustrates the clearing of reads for processing, in accordance with aspects described herein;
FIG. 3 depicts an example method for processing an initial alignment, in accordance with aspects described herein;
FIGS. 4A and 4B depict example position maps for reads containing softclips, insertions, and deletions, in accordance with aspects described herein;
FIGS. 5A-5C depict the flattening of an aligned read, in accordance with aspects described herein;
FIGS. 6A-6D depict introduction of candidate indel(s) into a flattened aligned read, in accordance with aspects described herein;
FIG. 7 depicts one example of read realignment processing, in accordance with aspects described herein;
FIG. 8 depicts an example process for selecting a best candidate realignment, in accordance with aspects described herein;
FIG. 9 depicts example realign-to-targets processing, in accordance with aspects described herein;
FIGS. 10A and 10B depict example processes for left and right anchor realignment results, in accordance with aspects described herein;
FIG. 11 depicts an example process for adding an indel to obtain a resulting realignment, in accordance with aspects described herein;
FIG. 12 depicts a distribution of variant lengths used in a simulation analysis in accordance with aspects described herein;
FIG. 13 depicts possible outcomes of a truth variant evaluation in accordance with aspects described herein;
FIG. 14 depicts true and false positive rates for simulation BAMs generated by iSAAC with priors that are non-realigned, GATK-realigned, or realigned in accordance with aspects of a realignment method as disclosed herein;
FIG. 15 depicts true and false positive rates for simulation BAMs generated by iSAAC with priors that are non-realigned, GATK-realigned, or realigned in accordance with aspects of a realignment method as disclosed herein;
FIG. 16 depicts overall per-sample somatic mutation count for samples that are non-realigned, GATK-realigned, or realigned in accordance with aspects of a realignment method as disclosed herein;
FIG. 17 depicts per-sample somatic mutation count, broken down by mutation type, for samples that are non-realigned, GATK-realigned, or realigned in accordance with aspects of a realignment method as disclosed herein;
FIG. 18 depicts realignment time per million alignments for GATK and aspects of a realignment method in accordance with aspects described herein;
FIG. 19 depicts an example process for sequence alignment processing, in accordance with aspects described herein;
FIG. 20 depicts an example process for sequencing data read realignment processing, in accordance with aspects described herein;
FIG. 21 depicts an example process for determining eligibility of an initial alignment to undergo sequencing data read realignment processing, in accordance with aspects described herein;
FIG. 22 depict one example of a computer system and associated devices to incorporate and/or use aspects described herein;
FIG. 23 depicts one example of a sequencing device that may be used in conjunction with aspects described herein; and
FIG. 24 depicts one embodiment of a cloud computing environment in accordance with aspects described herein.

### DETAILED DESCRIPTION

The development of next generation sequencing technology (NGS) has transformed genetic sequencing, permitting generation of a high volume of copies of genetic sequences, such as from the genome of an organism, aligning these sequences to create a putative recapitulation of the sequence of nucleotides of the copied genetic sequences. By identifying the sequence of nucleotide base pairs in the aligned copies, the sequence of nucleotides in the original sequence may be determined. One use of such technology is for identification, understanding, prevention, treatment, or cure of disease. For example, NGS may be used to identify an individual's genomic sequences to identify whether she possesses nucleotide sequences believed to underlie or render susceptibility to particular diseases, or identify such sequences which may do so, or determine whether a given pharmacological or other treatment may be beneficial in treating disorders of such an individual.

The high volume of sequence information that must be processed in order to derive the sequence of nucleotides from alignment of copies thereof is, in many cases, massive. For example, there are approximately three billion base pairs in the human genome. The ability to determine such large nucleotide sequence requires advanced computer processing technology. For example, synthesizing many copies of somewhat overlapping and/or adjoining portions of a large set of genetic sequences (for example, the billions of nucleotides in an entire reference genome, the tens or hundreds of millions of nucleotides in a chromosome or chromosomes, or long portions of a chromosome or other genomic sequence) via high throughput processing, and subsequently aligning them with one another to recapitulate, and identify the sequence of nucleotides of, the copied sequence typically requires processing of high volumes of data by a computer.

In many cases, errors may occur, leading to inaccurate representations of a genomic sequence in an alignment created thereof. A significant component of NGS technology includes a capability to identify and correct such errors. Where large genetic sequences are being sequenced, the number of potential errors may consequently be large as well. Computer technology is therefore desired to identify where such potential errors are, determine whether or not they are errors, and, if they are, what the correct sequences should be, often requiring selecting between multiple possibly correct sequences. Because of the potentially large number of such potential errors across huge spans of genetic sequences, automated processing of identifying and rectifying such errors as a component of the computer processing employed in NGS is highly desirable.

For example, the sequence of nucleotides within a chromosome possessed by most of the population may be known. An individual's sequences may then be determined and compared to such known sequences. Differences between the individual's sequences and the known sequences may be of importance, medically, genealogically, or otherwise. However, the presence of errors or potential errors in the aligned sequence determined for the individual by NGS complicates the identification of differences between the individual's genetic sequence and a known sequence, such as if an error is present but unidentified, or a difference between the individual's sequence and the known sequence is erroneously not detected. The present disclosure includes computer technology for improving automated identification and correction of certain types of errors that may occur in NGS and the associated informatics processing used to produce sequence alignments. Advantages include decreased processing time and increased identification and correction of errors, thereby improving usability of NGS tools and related technology.

Specifically, aspects described herein address problems of false positive (often single nucleotide variant) and false negative (often indel) variant calls caused by improper alignment of indel-containing sequencing data reads to a reference genome. Processes described herein may realign reads in a way that honors the existing representation of true indels and rejects low-frequency "noisy" variants, all in a short running time. Generally, one or more reads, or read sequences, may correspond to a position in a genetic sequence being sequenced by NGS. With the production of many reads spanning, in aggregate, all positions of a sequence being sequenced and aligning them in order from positions corresponding to one end of the sequence being sequenced to the other and identifying the order of nucleotides represented thereby, the complete sequence may be determined. As each read or reads corresponding to a position in the genetic sequence being sequenced are identified as corresponding to that position, they may be considered as having been aligned, or an aligned read. Errors may arise, however, in identifying, or calling, the presence of indels in an alignment, owing to difficulties in accurately identifying with certainty indels indicated by aligned reads.

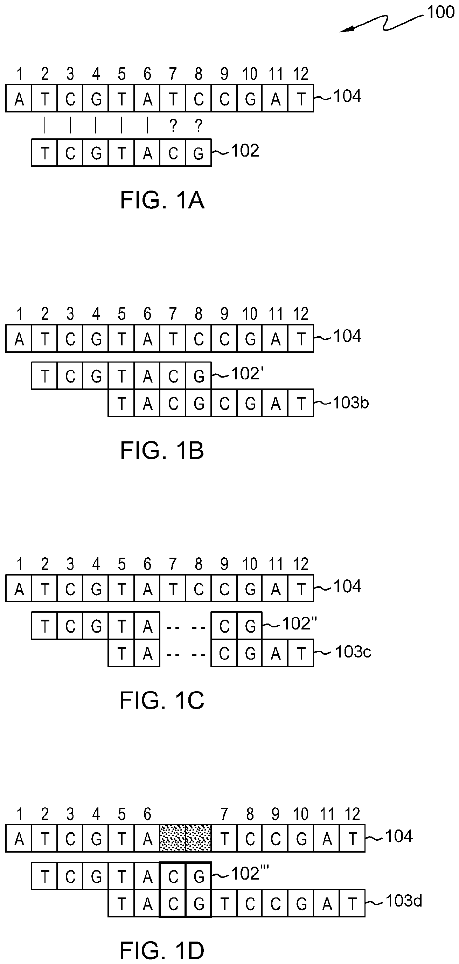

The lack of a two-sided context presents a challenge to the accurate calling of indels. When calling indels, a two-sided context may help indicate where the variation begins and ends. FIGS. 1A-1D illustrate how a two-sided context can be used to explain varying bases toward an end of a read. FIG. 1A depicts an initial alignment 100 of a read sequence 102 (also referred to herein as a "read") to a reference sequence 104 (also referred to herein as a "reference"), giving rise to an aligned read. In practice, a "read" and a "reference" may actually be a portion of a longer sequence of nucleotides that may also be referred to as a read sequence and the reference sequence. Nucleotide base positions 1 through 12 are noted above the reference sequence 104. Aligned read 102, which is seven nucleotides long in this example, matches to a portion of reference 104 for the first five bases of read 102. That is, the sequence T-C-G-T-A in base positions 2 through 6 match as between the aligned read 102 and the reference 104. The sequences diverge beginning at base position 7, where sequence C-G is observed in the read sequence at positions 7 and 8. FIGS. 1B through 1C depict three alternative ways to explain this variation by illustrating the variant in different two-sided contexts. In FIG. 1B, further context provided by the downstream sequence (e.g. another aligned read 103b in this example) identifies the varying bases C-G in positions 7 and 8 as point mutations. In FIG. 1C, the further context provided by 103c indicates that the variation is explained by a deletion that is two bases long at positions 7 and 8. In FIG. 1D, the further context provided by 103d indicates that the variation is explained by an insertion that is two bases long between positions 6 and 7. Differences in two-sided contexts for a given read sequence aligned as is 102 may give rise to different read alignments (e.g., 102', 102", or 102‴) of said read sequence.

Sequencing data gathered as part of a sequence analysis is stored in a sequence alignment dataset. Common file types for storing sequence alignment data are the SAM (.sam) and BAM (.bam) file formats. Sequence alignment software ("aligners") outputs a sequence alignment dataset file, e.g. a BAM file, that indicates alignments of read sequence(s) to a reference genome and indicates evidence that indel(s) may be present in these aligned reads. Aligners will usually have higher penalties for opening "gaps" (indels) than they do for assigning mismatches, which becomes especially pronounced at the end of a read. As a result, many sequence variations may be mistakenly called mismatches or may be softclipped even when other read evidence indicates that there may be an indel present.

Aspects described herein reprocess a sequence alignment dataset file, taking information from reads aligned nearby as indicated in a source/original/input sequence alignment dataset to form a surrounding context. This approach collects existing indel observations from the initial alignments as indicated in the input sequence alignment dataset and processes them by attempting to realign imperfectly aligned reads around observed indels such that mismatches are minimized. In some examples, reads that are not initially indicated to contain any indels are realigned such that they do indicate an indel relative to the reference. There may initially be little evidence in the sequence alignment dataset that a particular read contains any indels. However, aspects described herein can "rescue" the read when presence of an indel is more appropriately to be indicated by a realignment. By way of specific example, it may be the case that only one read aligned to a region of the reference genomic sequence indicated in the input sequence alignment dataset reflects an indel but after processing the initial alignments as described herein, several reads, e.g. of an output sequence alignment dataset output by processes described herein, support an indel being present.

In addition to reducing false negatives as above, approaches described herein can also reduce false positives by eliminating some mismatches or some indels initially indicated in one or more reads of the input sequence alignment dataset.

Processes described herein present a local indel realignment algorithm. This can help minimize mismatches by realigning input reads around indels, such as those observed in the input sequence alignment dataset file and/or indicated in a reference indel dataset, such as a 'priors' Variant Call Format (.VCF) file. The VCF priors may be provided as an input to an algorithm and indicate assumed indels in the source sequence alignment dataset file.

At a high level, a computer system may receive as input an input sequence alignment dataset and execute an algorithm to read through the input dataset, collect existing indel observations, and process one or more initial alignments from the sequence alignment dataset by attempting to realign the read of each initial alignment around observed indels. The algorithm may provide a new, 'realigned' sorted indexed sequence alignment dataset, for instance as an output BAM or other sequence alignment dataset file. In cases where the realignment of the read to the reference is better than the initial alignment of the read to the reference, the realignment may be output in place of the initial alignment. Otherwise, the initial alignment may be output as-is from the input sequence alignment dataset. The output sequence alignment dataset may be another file separate from the original sequence alignment dataset, or may be a modified version of the input sequence alignment dataset, in which the algorithm may directly modify/overwrite the original sequence alignment dataset.

In particular examples, the algorithm steps through the input sequence alignment dataset collecting existing indel observations and adding them to a set of candidate indels for use in realignment processing for a particular initial alignment. Whether an observed indel is considered a candidate can depend on any desired parameter, such as the observed allele frequency of the indel. In some examples, a user-configurable threshold allele frequency is provided as a parameter or other input to the algorithm, for instance as a command line parameter or as a parameter specified as an option in a software setting. An observed indel that appears at least as frequently as the frequency indicated by the threshold may be considered a candidate indel. The frequency may include a total number of reads aligned to a given position in a reference sequence that indicate the presence of a given indel at said position. Or, the frequency may include a proportion, out of a total number of reads aligned to a given position in a reference sequence, that indicate the presence of a given indel at said position. The configurable threshold may be set as low as 1, indicating that appearance of the indel in only one read aligned to a given position of a reference sequence constitutes enough evidence for the indel to be considered a candidate. Or, the configurable threshold may be a pre-determined proportion, of between 0 and 1, of how many reads aligned to a given position in a reference sequence, out of a total number of such reads, indicating the presence of the indel constitutes enough evidence for the indel to be considered a candidate. In practice, noise and other considerations may dictate that the frequency be set to something higher. In addition, any indel provided in an optional priors VCF reference indel dataset may be considered as a candidate indel.

A computer system in reading through the sequence alignment dataset may proceed generally from a beginning to an end of the reference genomic sequence being mapped. Candidate indels relevant to an individual alignment may occur before or after that alignment's original position (i.e. upstream or downstream relative to the reference genomic sequence). Upcoming, to-be-processed, reads may provide further support to a candidate indel. Because of this, the algorithm may hold encountered initial alignments in memory until they are considered cleared for processing, rather than processing those initial alignments immediately without reading alignments of reads to locations further down the reference genomic sequence. Cleared alignments are those whose position as indicated in the sequence alignment dataset is upstream from an upstream end of a window, of configurable window size, past the end position of the alignment. This allows collection of candidate indels for a given read from the regions before and after that read. The genomic window size correlates to a number of bases past the initial alignment that must have been read before the algorithm is satisfied that it has collected the information that is deemed potentially relevant to the alignment. The window size may be configurable, for instance as a command line parameter. A larger window size allows for larger and more distant indels to be considered but performance of the computer system may be negatively impacted if the window size is set too large due to the greater demand on resources. In particular examples, window sizes of 250-1000 bases may be sufficient for general use.

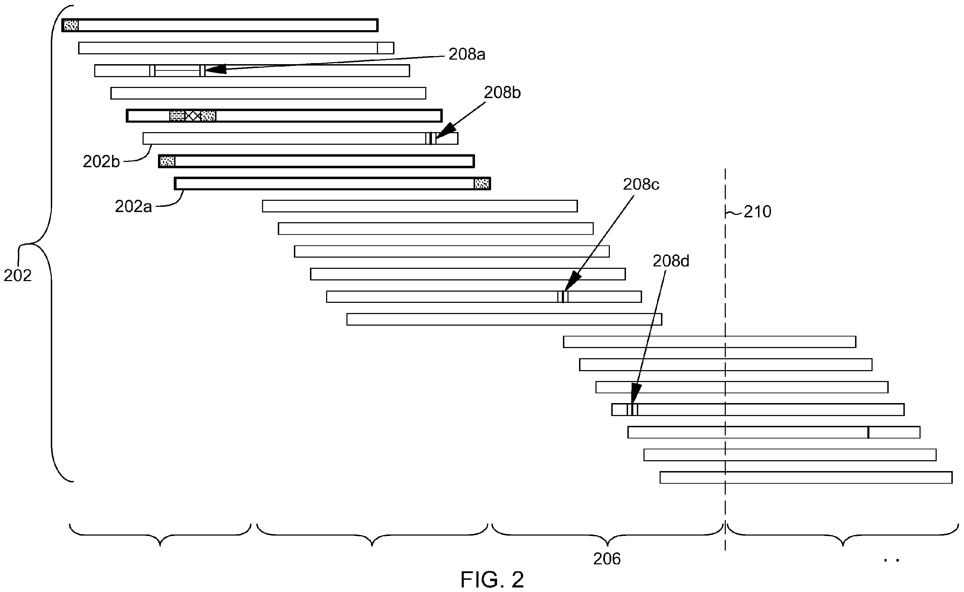

FIG. 2 illustrates the clearing of reads for processing in accordance with aspects described herein. The genomic block or window size is indicated by 206. Reads 202 are individually aligned in the horizontal direction (in this example) to a corresponding location relative to a reference genomic sequence (not shown). Indels 208a-208d are indels indicated in various reads. 210 denotes the point at which the first group of reads - the top-most 8 reads 202 in FIG. 2 - become cleared for processing. This is one window size from the end of the last read (202a) of that group. Delaying the alignment processing for a configurable window ensures that when processing an initial alignment of, e.g., read 202b, not only will indel 208b (which is part of the initial alignment) and upstream indel 208a be considered, but so too will downstream indels 208c and 208d because they are located within the window 206 upstream from point 210 at which the alignments of the first 8 reads become cleared to process.

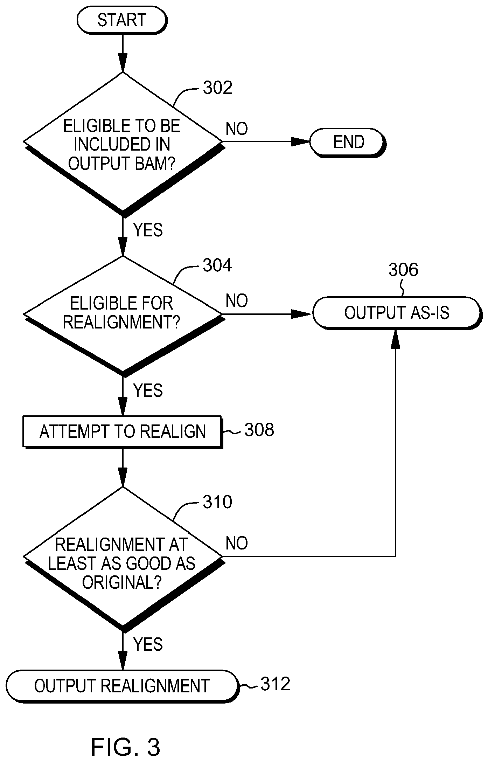

Initial alignments that are cleared for processing may undergo processing, an example method of which is described and depicted with reference to FIG. 3. The method of FIG. 3 is a process that may be performed by one or more computer systems. The process initially determines whether the alignment is eligible to be included in an output sequence alignment dataset (302), a BAM file in this example. In this regard, software performing the processing may have a configuration setting that enables processing to skip and remove certain alignments, such as PCR-duplicate alignments, so that these are ignored if that setting is enabled. If the initial alignment is ineligible for inclusion, the processing of the initial alignment ends without outputting the alignment. Otherwise, the processing continues by determining whether the initial alignment is eligible for realignment processing (304). Eligibility may be determined based on any desired factors. As examples, it may be determined (i) whether it is perfectly aligned, e.g. whether there are one or more bases mismatched as between the aligned read of the alignment and the reference sequence to which it is aligned, (ii) whether the aligned read includes a softclip, (iii) whether or not the initial alignment is a secondary alignment, and/or (iv) whether there are candidate indels around the aligned read in a region of bases of a reference genomic sequence indicated in the sequence alignment dataset. In one particular example, if the alignment is perfectly aligned, there are no softclips, it is a secondary alignment, or there are no candidate indels in the region, then the alignment is determined not to be eligible for realignment processing (304 - No) and the process outputs the alignment as-is (306), for instance by buffering it for direct output to the output sequence alignment dataset.

If instead at 304 it is determined that the alignment is eligible for realignment processing (304 - Yes), for instance if it is not perfectly aligned, there are softclip(s), it is not a secondary alignment, and/or there are candidate indels in the region, the process continues by attempting realignment processing to realign the initial alignment (308). Such realignment is described in further detail below as part of a read realignment procedure. This realignment procedure provides what is considered a "best" realignment. After the realignment processing, it is determined whether the best realignment is at least as good as the original initial alignment (310). If not, then the initial alignment is output as-is (306). Otherwise, the best realignment is output (312). Thus, in any case where an initial alignment is processed, an alignment of the given read to the reference may by output, the alignment being either the initial alignment (306) or the realigned alignment (312).

By the time an alignment is being considered for realignment (308), all candidate indels that were observed and could impact that read sequence of the alignment (including any from the original alignment itself, surrounding indels, and any "priors") have been collected to form a set of candidate indels that are candidates for introduction to provide a candidate realignment of the read to the reference. An iterative process is commenced that introduces each candidate indel (and in some examples combinations of two or more such candidate indels) into a flattened version of the aligned read. In some examples, the indel(s) are introduced from the left (i.e., from the upstream, or 5-prime, direction) and the right sides (i.e., from the downstream, or 3-prime direction) of the flattened aligned read. Each iteration provides a resulting `candidate realignment' that is evaluated to determine how good the realignment is. The evaluation may consider any desired indicator(s) of quality, for instance a number of mismatched bases between the aligned read of the realignment and the reference, number of indels, locations of indels, and/or number of softclipped bases as examples.

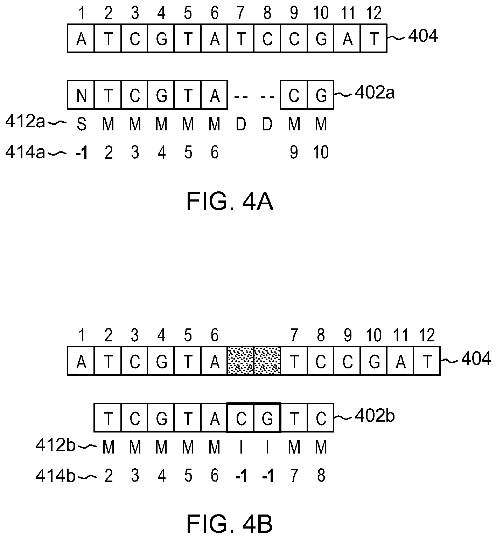

One concept described herein is a position map, which is an array of chromosomal coordinates for each base in the read. The position map is a data structure used to represent the sequences in the sequence alignment dataset. FIG. 4A depicts an example position map for an aligned read containing a softclip and deletion, and FIG. 4B depicts an example position map for an aligned read containing an insertion, in accordance with aspects described herein. Referring first to FIG. 4A, aligned read 402a is shown aligned with reference 404, corresponding CIGAR operations 412a are shown below the read. Below the CIGAR operations 412a is the position map 414a. The position map generally mirrors the base positions shown above reference 404 except that softclipped or inserted bases, which do not map to the reference genome, are given a position of "-1" in the position map, and deleted bases (shown in positions 7 and 8) are absent from the read so they do not have their own position. Instead, a deletion is evident by a jump in position between two consecutive read bases, for example a jump from 6 to 9 as shown in position map 414a indicates a 2 base pair (bp) deletion. Thus, FIG. 4A depicts an example position map for a read reflecting a chrN:2 (1S5M2D2M), with a softclip and a 2bp deletion. The softclip indicated in FIG. 4A is an N-type softclip. The initial aligner has softclipped part of the read, assigning it an "N", and indicating that it is unable to tell what the base is. "N" is a particular type of softclip; other types of softclips may have a base identified but still be considered a softclip.

FIG. 4B depicts another example position map 414b for a read reflecting a chrN:2 (5M2I2M) with a 2bp insertion (between positions 6 and 7). The insertion is shown in reference 404 and reflected in the aligned read 402b, CIGAR operations 412b, and position map 414b.

Read realignment may involve manipulation of the position map and subsequent comparison of the nucleotide-position pairs to the reference genome. Each aligned read being realigned may first be stripped of its existing indels and non-N type softclips to create a "blank slate". This provides a read that effectively starts with the assumption that it is indel-free. The indel-free read is referred to herein as a "flattened" read sequence, or flattened aligned read - a flattened version of the read in the initial alignment. Candidate indels are then iteratively introduced into the flattened aligned read and evaluated for agreement with the reference. This introduction may be accomplished by manipulating the position map. The resulting nucleotide-position pairs may then be compared to the reference genome.

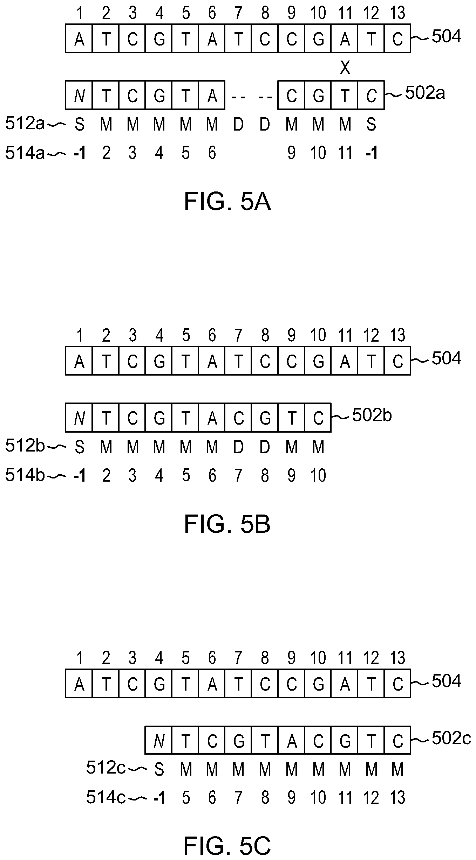

FIGS. 5A-5C depict the flattening of an aligned read, in accordance with aspects described herein. FIG. 5A shows the initial aligned read 502a aligned as indicated relative to the reference 504 and showing the corresponding CIGAR operations 512a. Position map 514a indicates an N-type softclip in position 1 and 2bp deletion in positions 7 and 8. FIG. 5B shows a flattened aligned read 502b, left anchored, meaning flattened to shift bases to the left (i.e., upstream, or in the 5-prime direction). CIGAR operations 512b and corresponding position map 514b have been updated as indicated. FIG. 5C shows a flattened aligned read 502c, right anchored, meaning flattened to shift bases to the right. CIGAR operations 512c and corresponding position map 514c have been updated as indicated in FIG. 5C.

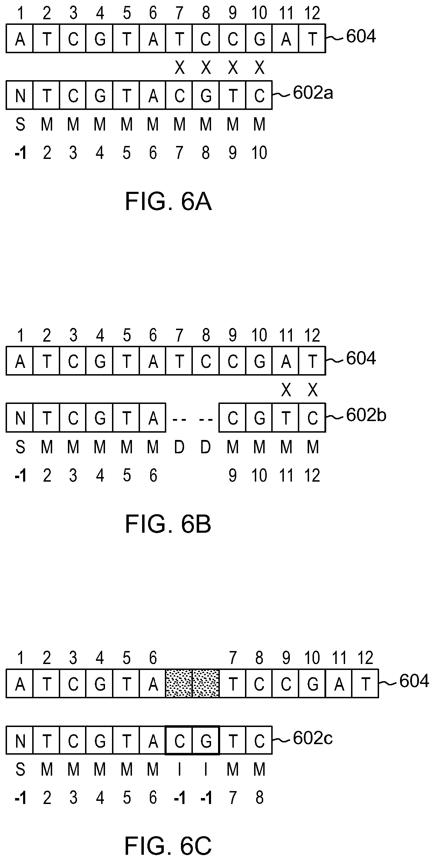

FIGS. 6A-6D depict introduction or 'injection' of candidate indel(s) into a flattened aligned read. The candidate indels may be those discovered within genomic proximity of the read being processed, along with any 'priors' indicated by a reference indel dataset if being used. In the examples of FIGS. 6A-6D, the proximal candidate indels include: chrN:6 ATC>A, chrN:6 A>ACG, and chrN:10 GA>G.

FIG. 6A depicts a candidate realignment with the realigned read 602a, flattened and left-anchored, aligned with reference 604. No indels are present, and the result is four bases mismatched as between the realigned read 602a of the candidate realignment and the reference sequence 604 - see positions 7-10.

FIG. 6B depicts another candidate realignment with the realigned read 602b having the chrN:6 ATC>A deletion indel introduced in positions 7 and 8. The last 4 bases of the read, C-G-T-C, are shifted down by 2 position in order to introduce that deletion. The result is two bases mismatched as between the read sequence 602b of the candidate realignment and the reference sequence 604 - see positions 11 and 12.

FIG. 6C depicts yet another candidate realignment with the realigned read 602c having the chrN:6 A>ACG insertion indel added between positions 6 and 7. The result is no bases mismatched as between the read sequence 602c of the candidate realignment and the reference sequence 604. As described further below, when such a candidate realignment is determined, the iteratively introducing the indels into the flattened aligned read may break to return that candidate realignment on account of the candidate completely matching the reference, which would considered a perfect alignment.

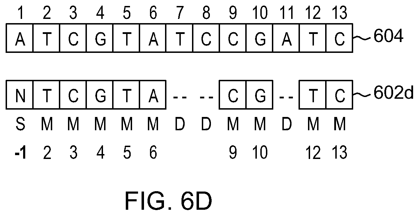

FIG. 6D depicts a candidate realignment with the realigned read 602d having the chrN:6 ATC>A deletion indel added in positions 7 and 8 and the chrN: 10 GA>G deletion indel added in position 11. This example illustrates an injection of two indels into the flattened aligned read. The result here too is no bases mismatched as between the read sequence 602d of the candidate realignment and the reference sequence 604. FIG. 6D is the same alignment as FIG. 6B but with an added indel.

One goal in finding the desired realignment may be to prioritize minimizing mismatches and then minimizing the number of indels to reach the best realignment. A realignment with a single indel and no mismatches may be considered to be as good as it gets, in which case realignment processing for that alignment may cease and return the realignment. This may then be compared to the initial alignment to determine which is the better alignment to be output. Alternatively, when a 'perfect alignment' is not encountered during the realignment processing, the `best' candidate realignment from the combinations considered may be compared to the original alignment, and the better selected for output as described below.

When determining the "best" candidate alignment, rules may be used and applied in an order or priority. In some examples, the current best candidate realignment is stored and compared with a next determined realignment candidate. The two are compared according to the rule(s) and if the realignment candidate is better, it is prioritized as the new best candidate realignment, replacing the old candidate realignment. An example of such rules and prioritization is:
(i) If an alignment of a read has just a single base mismatched to the reference and no indels, while another alignment of the read has one or more indels, prefer the alignment having the single mismatch and no indels. An alignment having no indels and only a single base mismatched is prioritized over a candidate alignment having one or more indels;
(ii) Minimize number of bases mismatched (i.e. mismatches between the aligned read of the alignment and the reference), regardless of number of indels. An alignment having a lesser number of bases mismatched is prioritized over an alignment having a greater number of bases mismatched;
(iii) Given the same number of bases mismatched to the reference, prefer the alignment with fewer non-N softclips. As between alignments having a same number of bases mismatched, an alignment having a lesser number of softclips of a specified type (e.g. N) is prioritized over an alignment having a greater number of softclips of the specified type; and
(iv) Given the same number bases of mismatched to the reference, prefer the alignment with fewer indels. As between different alignments having a same number of bases mismatched, an alignment having a lesser number of indels is prioritized over an alignment having a greater number of indels.

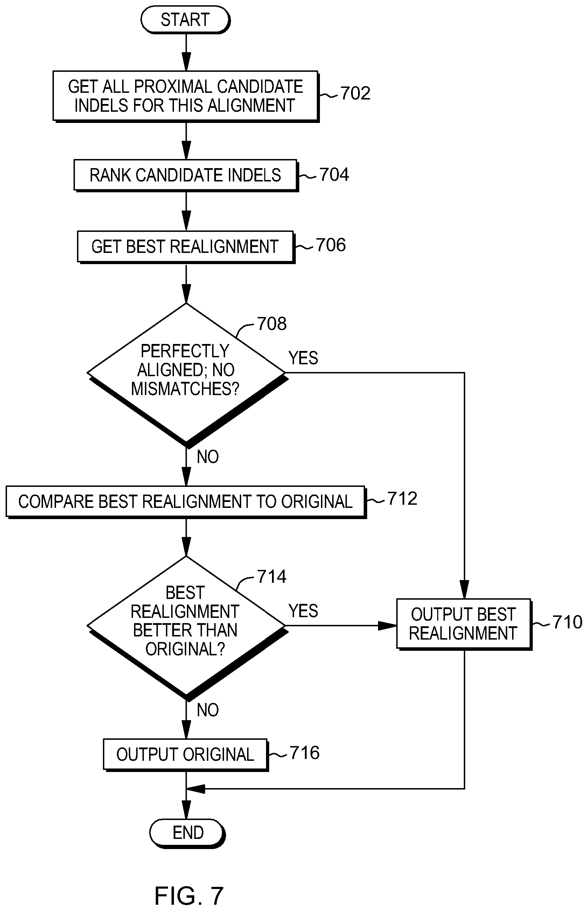

The input sequence alignment dataset may be processed by a computer system to read the data alignment-by-alignment. These initial alignments are read into memory and each initial alignment is eventually cleared for processing based on a sliding window as described above. If this processing determines the cleared initial alignment is eligible for realignment processing, then read realignment processing as described and depicted with reference to FIG. 7 is performed for each initial alignment cleared for processing. The processing of FIG. 7 may be performed by one or more computer systems.

The process begins by getting all proximal candidate indels for this originally aligned read, i.e. observed indels in the region (702). Proximal indels may be those that are within the region or window considered relevant to this read alignment, and thus may be any indels seen in any of several different alignments indicated in the sequence alignment dataset. This set of indels, optionally together with indel(s) indicated in a reference indel dataset as `priors', or known/assumed-to-be present indels, forms the set of candidate indels.

The process then ranks these candidate indels (704) relevant to the initial alignment. This ranking or prioritization may be based on any desired rule(s), examples of which are as follows and applied in the following order:
(i) "Known"/priors first (if using) - the prioritizing can prioritize an indel indicated by a reference indel dataset to be a prior known indel over an indel not indicated by the reference indel dataset to be a prior known indel even if that indel not indicated as a "prior" is heavily indicated in the sequence alignment dataset;
(ii) Larger/longer indels first - the prioritizing can prioritize an indel of longer length over an indel of shorter length. Longer indels may be ranked higher than smaller ones, even those that may appear more frequently in the sequence alignment dataset;
(iii) Higher frequency first, e.g. indel present in a higher number of reads indicating presence of the indel at the given position - the prioritizing can prioritize an indel indicated in a greater total number of read sequences, or greater proportion thereof, of the sequence alignment dataset over an indel indicated in a lesser number or proportion, respectively, of aligned reads of the sequence alignment dataset corresponding to a given position in the reference sequence;
(iv) If same frequency, left-most indel first - the prioritizing can prioritize, as between different indels indicated in a same number of aligned reads of the sequence alignment dataset, an indel whose location relative to a reference genomic sequence indicated by the sequence alignment dataset is upstream from a location, in the reference genomic sequence, indicated for another indel. As an example, indel 208a may actually be ranked higher than indel 208b when processing attempting to realign read 202b.

The ranking is an indication of which indels are weighted heavier in terms of the probability of their presence as compared to other indels. If two possible candidate indels could have provided two different candidate realignments with a same number (zero or more) of bases mismatched, the prioritization indicates which indels are to be more heavily trusted. The example priority rules above push toward the known, longer, more frequently occurring indels. The prioritization reflects the more likely true indels present.

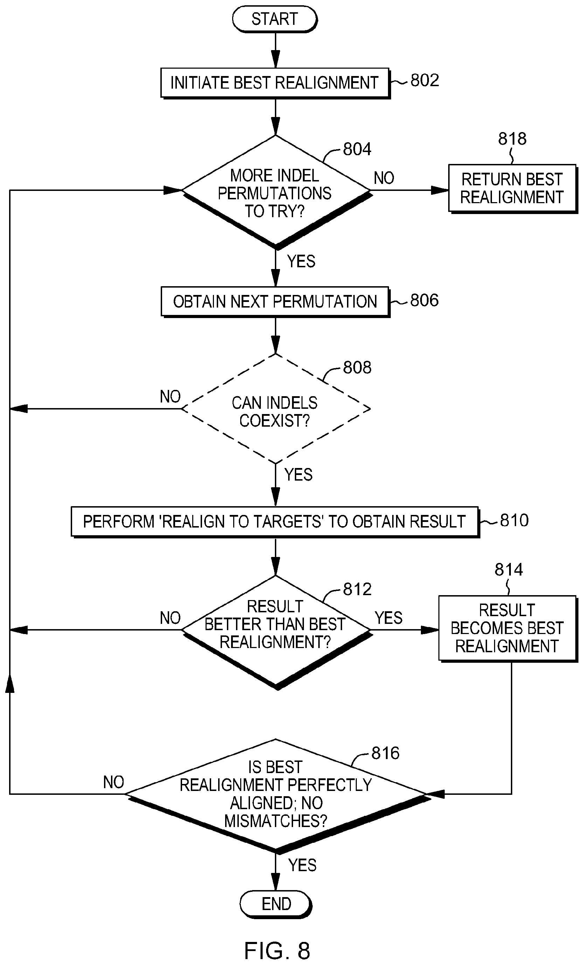

The process of FIG. 7 continues by getting a `best' realignment (706), described in further detail with reference to FIG. 8. The best realignment is the result of stepping through candidate indel(s) and introducing them iteratively into a flattened version of the aligned read of the original alignment to produce candidate realignment(s). The iterating through the ranked candidate indels realigns the read with permutations of one indel, two indels, and so forth up to and including n number of indels. In some embodiments, n is 3. Each iteration produces a candidate realignment. The `best' of these realignments may be selected using selection criteria. One goal may be to achieve as few mismatches as possible between the modified read, having injected indel(s), and the reference.

Continuing with the process of FIG. 7, after getting the best candidate realignment, the process determines whether the read, as realigned, is perfectly aligned to the reference (708), i.e. whether the aligned read of the candidate realignment, the aligned read having one or more indels introduced therein, aligns with the reference sequence with no bases mismatched as between the aligned read of the realignment and the reference. An example of this is depicted in FIG. 6C. With the C-G insertion indel between positions 6 and 7, the aligned read 602c perfectly aligns with the reference 604. If the selected best realignment presents a perfect realignment (708 - Y), then the process outputs that selected best realignment 710 in place of the original alignment from the input sequence alignment dataset.

Otherwise, if there are bases mismatched as between the realigned read of the best candidate realignment and the reference (708 - N), the process proceeds by comparing the best candidate realignment with the original alignment (712). Ultimately the goal is to output the better alignment of the two. Thus, based on the comparison, the process determines whether the best candidate realignment provided by 706 is better than the original alignment (714). If so, the process outputs this best realignment (710). In a particular example, if the best candidate realignment is better than or as good as the original alignment, a mapping quality is adjusted, if appropriate (e.g. set to 40 if the original quality was 20 or lower and the realignment has no mismatches), and the process outputs the best candidate realignment to an output sequence alignment dataset after this mapping quality adjustment. Returning to inquiry 714, if the best candidate realignment is not better than or as good as the original alignment (714 - N), then the process outputs the original alignment (716).

The selection criteria for selecting the better of the original alignment and the best candidate realignment may be the same or different than the selection criteria applied to determine the best of the realignment candidate from 706. In particular examples, the selection criteria for selecting the best candidate realignment and/or the better of the best candidate realignment and the original alignment may be based on: number of bases mismatched as between the aligned read of the alignment and the reference sequence, number of indels indicated by the alignment, location(s), corresponding to the indel(s), in the reference genomic sequence indicated in the sequence alignment dataset, and/or number of softclipped bases indicated by the alignment. "Alignment" in the foregoing encompasses both alignment (as in original alignment) and realignment (as in a candidate realignment), since both cases present an alignment of the corresponding read to the reference sequence.

As an example, the selection criteria can prioritize one or more of: an alignment having no indels and only a single base mismatched (as between the read and the reference of the alignment) over an alignment having one or more indels; an alignment having a lesser number of bases mismatched over an alignment having a greater number of bases mismatched; as between alignments having a same number of bases mismatched, an alignment having a lesser number of softclips of a specified type, such as N, over an alignment having a greater number of softclips of the specified type; and/or as between different alignments having a same number of bases mismatched, an alignment having a lesser number of indels over an alignment having a greater number of indels.

FIG. 8 depicts an example process for selecting a best candidate realignment, in accordance with aspects described herein. The processing of FIG. 8 may be performed by one or more computer systems. At a high level, the process introduces permutations of one or more indels into a modified, e.g. flattened, version of the aligned read of the initial alignment that is undergoing the realignment processing. Each introduction produces a candidate realignment. The process first introduces each indel individually into the flattened aligned read, providing candidate realignment(s), then introduces each combination of two indels into the read to provide additional candidate realignments. This may repeat for 3, 4, etc. indels until some configurable threshold is met. In some examples, this threshold is met after the introduction of permutations of 3 indels into the read. The priority in which the indels are introduced in the realignment processing follows the ranking of those indels as previously described. Also, in some examples, the processing is configured to break (exit/halt) whenever a perfect alignment has been determined.

By way of specific example, assume there are n candidate indels [I1, I2, I3, ..., In} ranked in order of priority, and the iterating proceeds through combinations of 1, then 2, then 3 indels. The iteratively introducing the indels into the flattened aligned read will proceed in the following order, with each iteration providing a candidate realignment:
- [Iterations of one indel:] Introduce I1, then 12, then 13, ..., then In; then
- [Iterations of two indels:] Introduce I1 + 12, then I1 + I3, ...., then I1 + In, then I2 + 13, then I2 + 14, ..., then I2 + I*n,* ..., then *In-1* + In; then
- [Iterations of three indels:] Introduce I1 + I2 + 13, ..., then In-2 + In-1 + I*n.*

The introduction of the indels injects the indel(s) into the flattened aligned read and checks how the modified read alignment lines up with the reference genome, which checking may be assisted by the modified position map produced.

As noted, if at any point during the iterating a candidate realignment that perfectly aligns to the reference is provided, the processing can break and select that candidate as the best candidate realignment to provide (FIG. 7, #706).

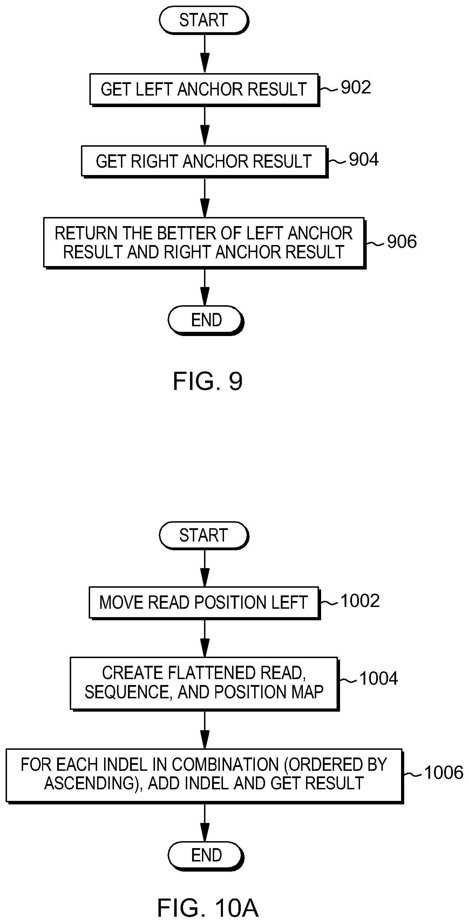

Referring to FIG. 8, the process begins by initiating the best realignment. In an example, this is initially null or defaulted to the original alignment as a placeholder but as processing of FIG. 8 continues will be replaced with the current best realignment encountered when processing the subject original alignment. The process enters a loop that begins by determining whether there are more indel permutations to try (804). If there are, the process obtains/identifies the next permutation to try (806). Then, an optional determination 808 is made when the next permutation to try includes multiple indels. Some indels may not coexist, in which case introducing them into the flattened aligned read to provide a candidate realignment does not make sense. Determination 808 may not be made during the initial iterations when only a single indel is introduced. Eventually if realignment processing reaches permutations of 2 or more indels then determination 808 may be made at each iteration. If at 808 it is determined that the indels up for introduction into the flattened read cannot coexist, the process proceeds to the next iteration by returning to 804 to determine whether there are more indel permutations to try. Otherwise, or if determination 808 is not performed because there is only a single indel being considered in the present iteration, the process proceeds by performing a 'realign-to-targets' process to obtain a result (810). This process is described in further detail with reference to FIG. 9.

The result obtained from 810 is a candidate realignment. The process of FIG. 8 then determines whether that result is better than the current best realignment (812). If so, the result becomes the new current best realignment (814). In one example, the result replaces the previously stored best realignment, which is discarded. Because the result was determined to be better than any prior-obtained candidate realignment in this processing, the process proceeds by determining whether the result - the new best realignment - is a perfect alignment, i.e. there are no bases mismatches as between the aligned read of the realignment and the reference (816). If so, the process ends and the best realignment is used as the selected best realignment. In some examples, this is output to an output sequence alignment dataset as the best alignment (FIG. 7, 710).

If at 816 is it determined that the new best realignment is not a perfect alignment, or if at 812 it is determined that the result obtained was no better than the current best realignment, the process returns to 804 to determine whether there are additional indel permutations to try. If not, then the process returns the current best realignment (818). It is seen that this process continues iterating until there are no more indel permutations to try (804 - No), or a perfect alignment is provided by candidate realignment that is determined (816 - Yes).

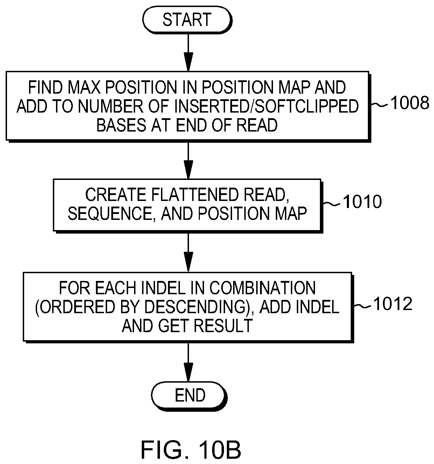

FIG. 9 depicts an example 'realign-to-targets' (810 of FIG. 8) processing in accordance with aspects described herein. The processing of FIG. 9 may be performed by one or more computer systems. The process gets a left-anchor result (902), described with reference to FIG. 10A, gets a right-anchor result (904), described with reference to FIG. 10B, and returns the better of the two (906). The selection criteria to select between the two may be any selection criteria desired, such as selection criteria described above. In a modified example, instead of obtaining a candidate realignment from both the left and right anchor processing, the realignment provided by the left anchor result is processed through FIG. 8 (812-816) and the right anchor result processing of FIG. 10B is performed only if it is determined that the left anchor result did not result in a perfect alignment (816 - No).

FIGS. 10A-10B depict example processes for left and right anchor realignment results, in accordance with aspects described herein. The processing of FIGS. 10A and 10B may be performed by one or more computer systems. The anchoring from the left or right is a reflection on which side of the read is assumed to be more accurate in terms of its identified bases. If one end of the read is trusted more than the other, the read is anchored from that end and the injection of the indel(s) proceeds from that end. In a left anchor realignment, the left side is trusted more than the right side. For the left anchor result processing, FIG. 10A, the process gets an adjusted start position by moving the read position left (1002) by the length of any prefix softclip (except for N-softclips, in some examples). If the read begins with (i) an insertion or (ii) softclip and insertion, the read position is shifted left by the length of that insertion. The anchor corresponds to the outermost matched nucleotide. The process then creates the flattened read, sequence, and position map (1004). An example is depicted in FIG. 5B. Then, for each of the one or more indels in the current permutation combination, and in rank order, the process adds the indel to obtain a resulting realignment (1006). FIG. 6C shows an example of one indel introduced into the flattened left-anchored read, and FIG. 6D shows an example of two indels introduced into the flattened read.

For the right anchor result processing, FIG. 10B, the process gets an adjusted end position by finding the maximum position in the position map and adding the number of inserted/softclipped bases that exist at the end of the read (1008). The adjusted start position of the read will be that maximal position minus the length of the read, not including N-type softclips. The process then creates the flattened aligned read, sequence, and position map (1010). An example is depicted in FIG. 5C. Then, for each of the one or more indels in the current permutation combination, and in order from right-ward (or upstream, towards the 5-prime end) to leftward (or downstream, towards the 3-prime end), the process adds the indel to obtain a resulting realignment (1012). For instance, if there are three indels to be introduced, the process introduces them from right to left, adding first the upstream-most indel of the three, then the upstream-most of the remaining two, then the third.

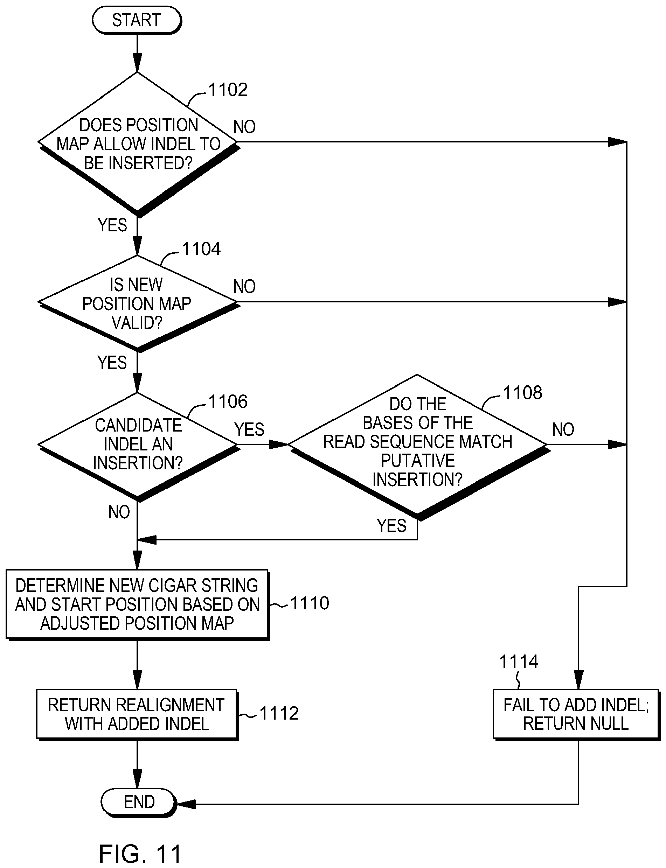

The `add indel and get result' processing (1006 of FIG. 10A, 1012 of FIG. 10B) is described with reference to FIG. 11. The processing of FIG. 11 may be performed by one or more computer systems. This is performed for each indel to be added. In cases where there are multiple indels to be added, a realignment resulting from the addition of an indel is what is modified with the addition of the next indel (layering on each consecutive indel to the resulting realignment). The candidate realignment that is ultimately produced from the processing of FIG. 10A or 10B and returned by 810 of FIG. 8 is the final realignment from performance of FIG. 11 to add each of the indel(s) for the combination.

The process of FIG. 11 assumes some starting realignment candidate, which would initially be the flattened read with no indels introduced but is replaced with an updated resulting realignment as each indel is added. The process begins by determining whether the position map allows an indel to be introduced (1102). If not, such as if, for example, the reference position of the indel to be introduced is off the position map, or is the final position in the position map, there is a failure to add the indel and the process returns NULL (1114) or some other desired result, then ends.

If the position map allows the indel to be introduced, the process determines whether the new position map (with the indel inserted) is valid (1104). If not, then there is a failure to add the indel and the process returns NULL (1114) or some other desired result, then ends. Otherwise, the process proceeds by determining whether the candidate indel is an insertion (1106). If so, it is determined whether the bases of the read sequence match the putative insertion (1108). The bases of the read sequence may match the putative insertion if the bases in the read sequence at the position of the putative insertion are the same bases as those specified in the putative insertion. As an illustrative example, if the following read sequence ATCTGA were anchored at position 10 (i.e. the 5-prime A is at chrN:10), and the putative insertion is chrN:12 C > CTG, it would be considered a match, because the next two bases in the read sequence after the C at chrN:12 are TG. By contrast, if the putative insertion is, as another illustrative example, chrN:12 C > CAA, it would not be a match because the next two bases in the read sequence after the C at chrN:12 are not AA. If the bases of the read sequence do not match the putative insertion, then there is a failure to add the indel and the process returns NULL (1114) or some other desired result, then ends.

If instead at 1108 it is determined that the bases of the read sequence match the putative insertion (1108 - Yes), or if it was determined at 1106 that the indel is not an insertion, e.g. it is a deletion, then the process proceeds by determining a new CIGAR positions string and start position based on the adjusted position map (1110). It then returns the resulting realignment with the indel added (1112) and ends.

Pseudocode is provided below for an example GetBestAlignment Routine (corresponding to FIG. 8), and example sub-routine RealignToTargets (corresponding to FIG. 9).

GetBestAlignment refers to the routine performed on the list of ranked candidate indels for introducing to the flattened read. Within this process, RealignToTargets is performed on each candidate indel both singly and in combination with other candidate indel(s). If at any point the introduction of a single indel results in a read with no mismatches, the process can exit with that realignment being considered the best realignment candidate. Otherwise, the process returns the "best" realignment, as measured by the rules/selection criteria described above from all assessed combinations of one to n indels, where n is the maximum number of indels to introduce.

GetBestAlignment Routine Pseudocode:
Initialize BestResultSoFar to empty;
For each candidate indel A, in order of ranking:
   // Try aligning to one indel:
   Perform RealignToTargets routine, get result ResultA;
   If ResultA is better than BestResultSoFar, ResultA becomes BestResultSoFar;
   If BestResultSoFar has 1 indel and 0 mismatches, break and keep that as best realignment.
   // Try aligning to two indels:
   For each additional candidate indel B:
      If the indels A and B can't coexist, skip this pair;
      Perform RealignToTargets routine, get result ResultAB;
      If ResultAB is better than BestResultSoFar, ResultAB becomes BestResultSoFar
   // Try aligning to three indels:
   If configured to try combination of three, for each additional candidate indel C:
      If the indels A, B, and C can't coexist, skip this threesome;
      If BestResultSoFar has > 0 mismatches:
         Perform RealignToTargets routine, get result ResultABC;
         If ResultABC is better than BestResultSoFar, ResultABC becomes BestResultSoFar;
Return BestResultSoFar;

RealignToTargets Routine Pseudocode:
Given: CombinationIndels, which is a list of, e.g., one to three candidate indels to be assessed in combination:
   // Get result using left anchor:
   Get adjusted position: Move the read position left by the length of the prefix softclip (except for N-softclips). If read begins with insertion or softclip + insertion, move the read position left by the length of that insertion;
   Create flattened read with a CIGAR, sequence, and position map (excluding terminal Ns) that assumes all matches. The resulting read would have a CIGAR string of "M" for every base in the read (excluding terminal Ns);
   Initialize ResultLeftAnchored;
   For each indel X in CombinationIndels, ordered by position ascending:
      Perform the AddIndelAndGetResult routine (FIG. 11), modifying ResultAlignment (layering on each consecutive indel);
   // Get result using right anchor:
      Get adjusted position: Find the maximum position in the position map, and add to it the number of inserted or softclipped bases that exist at the end of the read.
      The adjusted start position of the read will be that maximal position minus the read length;
      Create flattened read with a CIGAR, sequence, and position map (excluding terminal Ns) that assumes all matches. The resulting read would have a CIGAR string of "M" for every base in the read (excluding terminal Ns);
      Initialize ResultRightAnchored
      For each indel X in CombinationIndels, ordered by position descending:
         Perform the AddIndelAndGetResult routine, modifying ResultAlignment (layering on each consecutive indel);
Return the better of ResultLeftAnchored and ResultRightAnchored. In the case of a tie, return ResultLeftAnchored.

Aspects described herein can be used to adjust and improve sequencing data alignments output from an initial aligner. An aligner may output an initial sequence alignment dataset, which is provided as input to software configured to perform aspects described herein. The software outputs a sequence alignment dataset with realignments of one or more of the initial alignments.

The following presents a comparison of indel realignment according to aspects described herein (referred to below as Realigner) with indel realignment of the GATK Indel realigner offered by the Eli and Edythe L. Broad Institute of MIT and Harvard ("Broad Institute"), Cambridge, Massachusetts, USA.

A distinction of the Realigner lies in its ability to accurately realign reads around observed mutations and do so in less time than existing methods. To demonstrate this, Realigner was compared against the likely best-known local indel realigner in the bioinformatics community, the GATK Indel realigner (see, e.g. DePristo, M., Banks, E., Poplin, R., Garimella, K., Maguire, J., & Hartl, C. et al. A framework for variation discovery and genotyping using next-generation DNA sequencing data. Nature Genetics, 43(5), 491-498, (2011)) to determine whether it performed at least as well in a shorter amount of time.

Sensitivity and Specificity on Simulated Variant Data:

### Methods

To evaluate sensitivity, the following experiment was performed:
1. Simulate individual-variant FASTQ files of 200 insertions and 200 deletions of length 4-25bp (400 simulation FASTQs total).
2. Align simulated FASTQ files using the iSAAC aligner offered by Illumina, Inc., San Diego, California, U.S.A.. Two conditions were assessed: with and without `Priors'. Supplying a list of priors to iSAAC allows indels in the list to be favored over calling a string of mismatches at that position.
3. Realign each of the above conditions (with priors, without priors) using each of Realigner, GATK, and no realignment.
4. Call variants using the Pisces variant caller offered by Illumina, Inc.
5. Evaluate sensitivity and specificity of variants called.

### Samples used in the analysis:

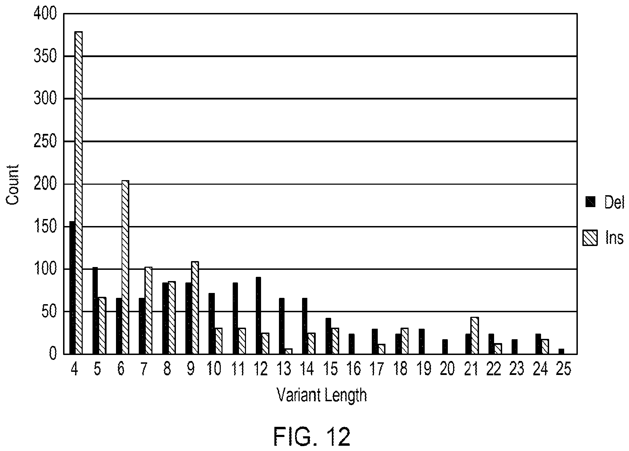

200 insertions and 200 deletions were randomly selected from a pool of ~2000 mid-length (4-25bp) indels. FIG. 12 depicts a distribution of variant lengths used in a simulation analysis in accordance with aspects described herein.

### Evaluation of Called Variants

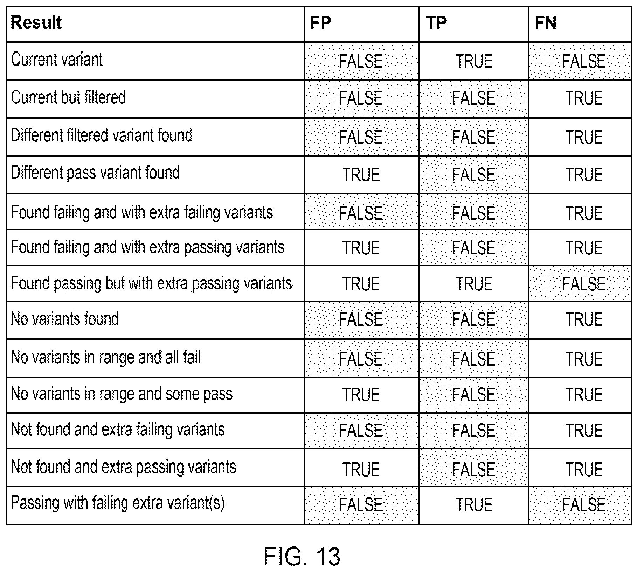

Each simulation sample is expected to have exactly one called variant. To evaluate the sensitivity and specificity of the outcome, all called variants are extracted from the VCF (yielding 0 to many variants, 0 to 1 of which will match the expected variant). The resulting variants are compared to the expected "truth" variant, resulting in one of the outcomes listed in FIG. 13, depicting possible outcomes of a truth variant evaluation in accordance with aspects described herein.

### Results

Using priors in the initial iSAAC alignment increased sensitivity for all conditions. Without realignment, 48.5% of variants were successfully called with no false positives. With GATK realignment, that portion rose to 48.8%, while Realigner achieved 75.3%. In all cases, if the variant was correctly called and passing, there were no other passing variants. In some examples, Realigner used with priors may produce fewer false negatives and fewer false positives than GATK realignment.

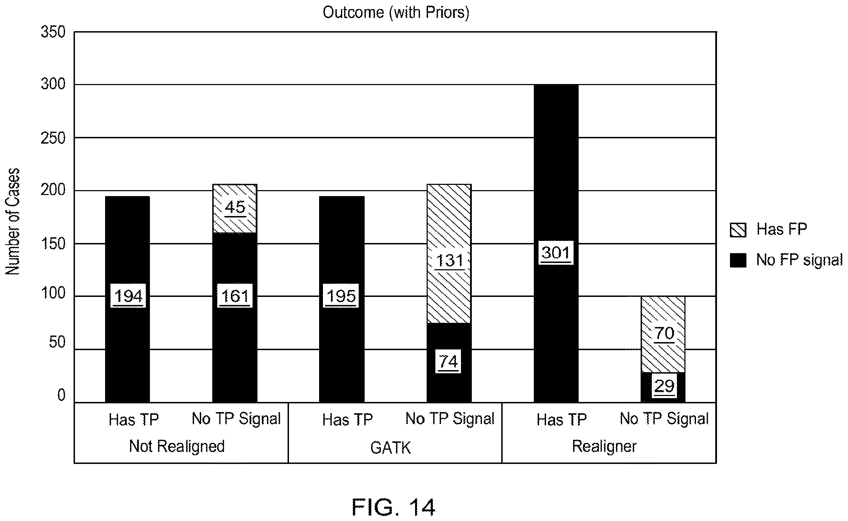

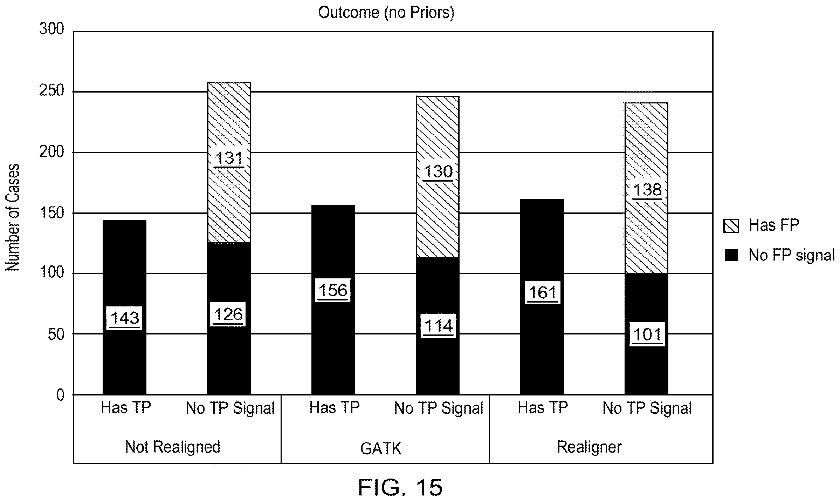

FIG. 14 depicts true and false positive rates for simulation BAMs generated by iSAAC with priors that are non-realigned, GATK-realigned, or realigned in accordance with aspects of a realignment method as disclosed herein. FIG. 15 depicts true and false positive rates for simulation BAMs generated by iSAAC with priors that are non-realigned, GATK-realigned, or realigned in accordance with aspects of a realignment method as disclosed herein. It should be noted that these results are based on specific desired representations of indels, which may not always be the left-aligned representation. GATK's representation of an indel will always be the left-most aligned, whereas Realigner maintains fidelity to the original representation of the indels it sees in the input BAM.

Specificity on FFPE Normal Samples:

### Methods

To evaluate specificity on realistic samples, normal (non-disease) samples were used. To sufficiently challenge the realigners, FFPE samples were used, which typically have poor DNA quality leading to a large number of low-frequency "noise" variants. In particular for Realigner, each of these low-frequency variants represents an opportunity to introduce a false variant.

Because these are normal, non-cancer samples, we assume that all true variants are at diploid frequencies (-50% for heterozygous, and -100% for homozygous variants). Thus, anything in the "somatic" range (<20% VAF) can be considered a false positive. Furthermore, the lower the resulting somatic mutation count, all other things being equal, the more accurate a realignment method may be considered to be.

### The following experiment was performed:

1. Run the iSAAC variant caller using a priors VCF containing the targeted variants from the Catalog of Somatic Mutations in Cancer (COSMIC) online database.
2. Realign the BAM files using Realigner or GATK.
3. Call variants using the Pisces variant caller.
4. Assess somatic mutation rate.

The analysis was conducted on 20 FFPE Normal samples, which were prepped and sequenced using the TruSight Tumor 170 assay offered by Illumina Inc., and had been processed through the TruSight Tumor 170 informatics pipeline up to the Alignment step.

### Results

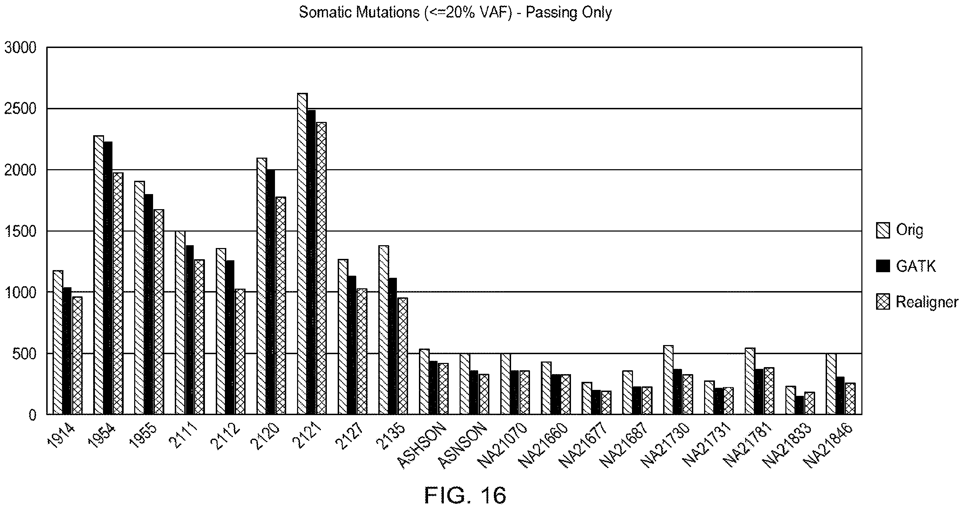

Realigner showed a lower somatic mutation rate (proxy for false positive rate in non-cancer samples) than either non-realigned or GATK-realigned results across the board (in only three of twenty cases did Realigner have a higher FP (false positive) rate than GATK, and all three were extremely close). Realigner appeared to have more aggressive deletion calling (see FIG. 16) than either non-realigned or GATK-realigned. In general, indel realignment significantly reduced false positives, and this was especially true for Realigner.

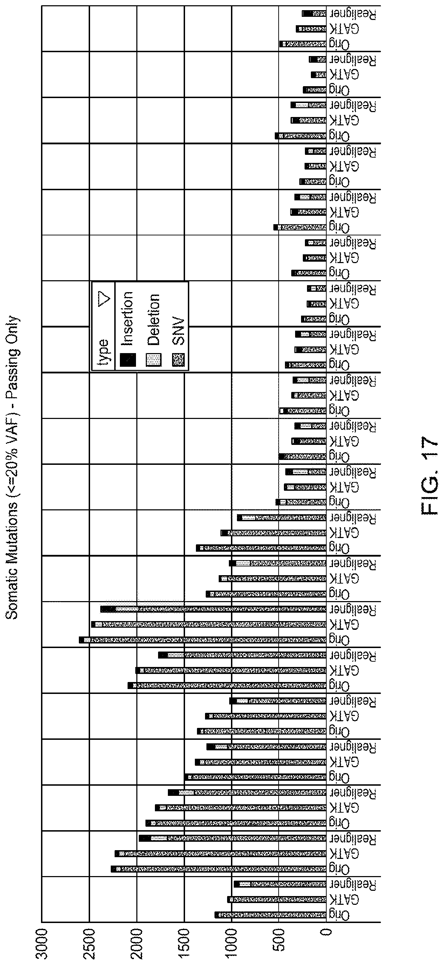

FIG. 16 depicts overall per-sample somatic mutation count (proxy for false positive count in non-cancer samples) for samples that are non-realigned, GATK-realigned, or realigned in accordance with aspects of a realignment method as disclosed herein. FIG. 17 depicts per-sample somatic mutation count (proxy for false positive count in non-cancer samples), broken down by mutation type, for samples that are non-realigned, GATK-realigned, or realigned in accordance with aspects of a realignment method as disclosed herein.

### Runtime Evaluation:

### Methods

The same 20 samples used for the FFPE Normal evaluation were assessed for compute time required to go from input BAM to realigned output BAM. The input BAM files each contained approximately 60 million reads.

### Results

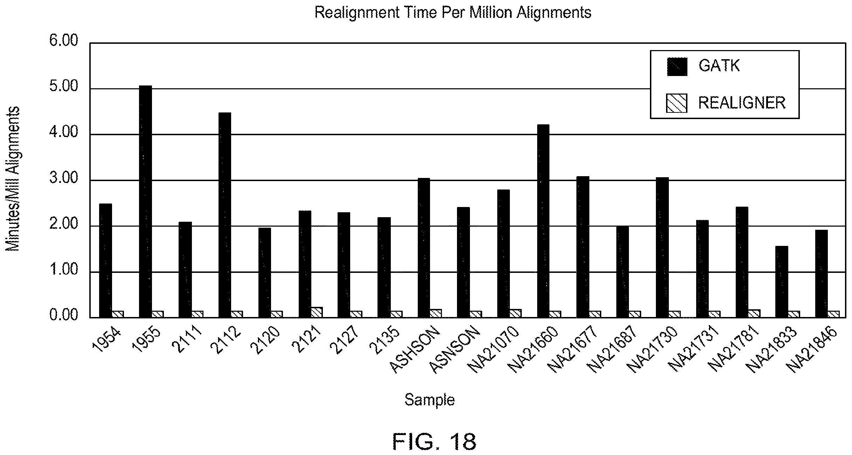

In all cases, Realigner was significantly faster than GATK on the mid-size BAMs. FIG. 18 depicts realignment time per million alignments for GATK and aspects of a realignment method in accordance with aspects described herein. Realignment time per million alignments ranged from about 1.5-5 minutes per million alignments for GATK, and was consistently under 10 seconds for Realigner, on the testing computer system(s).

The Realigner is a fast and accurate indel realignment algorithm that maintains fidelity to the representation of existing indels. It relies upon the presence of existing signal in the input sequence alignment dataset to realign around an indel. In the examples above, Realigner does especially well when used on BAM files that have been generated by iSAAC with consideration for priors, as this maximizes the likelihood that the input BAM will contain at least one read with the indel.

The expected gold standard for local realignment would involve a pileup approach with consensus generation and local realignment of the consensus. However, consensus-based solutions have been shown to be costly in terms of time and computing requirements. In contrast, the Realigner treats each read individually, using the context of proximal observed indels for a much simpler, candidate-based approach.

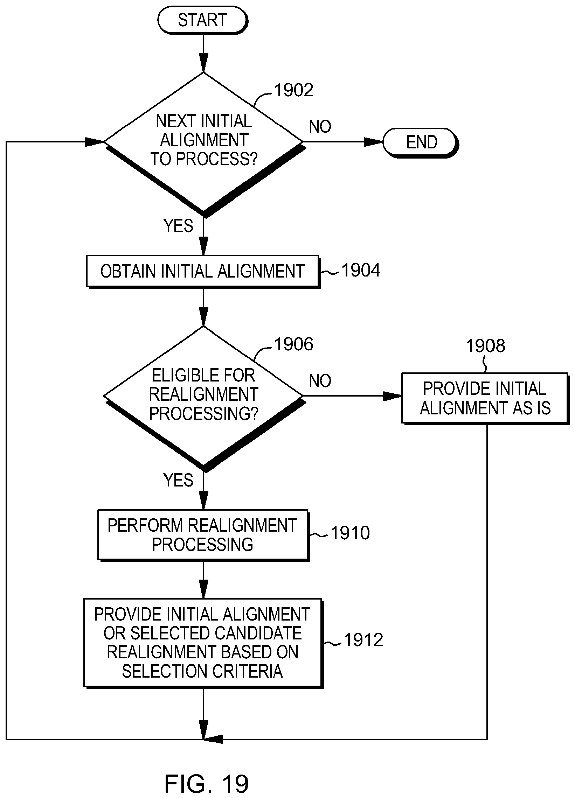

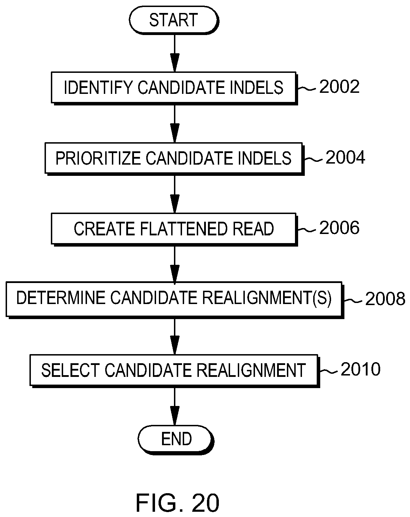

Accordingly, processes for sequence alignment processing are described herein. FIG. 19 depicts an example process for sequence alignment processing, in accordance with aspects described herein. The processing of FIG. 19 may be performed by one or more computer systems. In a particular example, software running on a computer system opens an input sequence alignment dataset file and reads its contents, which includes, as an example, binary representations of alignments of read sequences to reference sequence(s). The process begins by determining whether there is a next initial alignment to process (1902). If not, the process ends. If there is a next initial alignment to process, the process continues by obtaining from the sequence alignment dataset an initial alignment of a read sequence to a reference sequence (if not already read into memory) (1904). Processing is then performed on this initial alignment. Initially, the processing determines whether the obtained initial alignment is eligible for realignment (1906). If not, the process provides the initial alignment as is, absent performing the realignment processing (1908). Otherwise, if the initial alignment is eligible for realignment, then the process continues by performing realignment processing on the initial alignment (1910). The realignment processing realigns the read sequence to the reference sequence. Example realignment processing is depicted and described below with reference to FIG. 20. As part of that process, one or more candidate realignments are produced. The process of FIG. 19 then provides the initial alignment or a selected candidate realignment of the one or more candidate realignments based on one or more selection criteria (1912).

The selection criteria can be based at least in part on: number of bases mismatched, number of indels, location of indels relative to a reference genomic sequence indicated by the sequence alignment dataset, and/or number of softclipped bases. In some examples, the selection criteria prioritizes: an alignment having no indels and only a single base mismatched over an alignment having one or more indels for the providing; an alignment having a lesser number of bases mismatched over an alignment having a greater number of bases mismatched for the providing; as between different alignments having a same number of bases mismatched, an alignment having a lesser number of softclips of a specified type over an alignment having a greater number of softclips of the specified type for the providing; and/or as between different alignments having a same number of bases mismatched, an alignment having a lesser number of indels over an alignment having a greater number of indels for the providing.

Referring again to FIG. 19, after providing the appropriate alignment (1908, 1912), the process repeats by returning to 1902. This can repeat for each additional initial alignment of several, such as several that have become cleared for processing. Thus, the process repeats by performing processing for each additional initial alignment of one or more additional initial alignments. That is, the process repeats, for each additional initial alignment of one or more additional initial alignments of the sequence alignment dataset, the obtaining and the determining whether the obtained additional initial alignment is eligible for realignment.

FIG. 20 depicts an example process for realignment processing, in accordance with aspects described herein. The processing of FIG. 20 may be performed by one or more computer systems. The process begins by identifying one or more candidate indels (2002). The one or more candidate indels can be any in the aligned read, and potentially other indels aligned near or proximal to the aligned read. There may be zero or more indicated in the initial read alignment and zero or more near the aligned sequence, thus, the candidate indels can include zero or more indels in the aligned read and zero or more indels aligned proximal to the aligned read as indicated by the sequence alignment dataset. Additionally, and optionally, a reference indel dataset may supply one or more indels to the set of candidate indels for introduction.

The process of FIG. 20 then prioritizes the candidate indels (2004). The prioritizing prioritizes or ranks the candidate indels using any desired approach. For instance, the prioritizing prioritizes an indel indicated by a reference indel dataset to be a prior known indel over an indel not indicated by the reference indel dataset to be a prior known indel. Additionally or alternatively, the prioritizing prioritizes an indel of longer length over an indel of shorter length. Additionally or alternatively, the prioritizing prioritizes an indel indicated in a greater number of aligned reads of the sequence alignment dataset over an indel indicated in a lesser number of aligned reads of the sequence alignment dataset. Additionally or alternatively, the prioritizing prioritizes, as between different indels indicated in a same number of aligned reads of the sequence alignment dataset, an indel whose location relative to a reference genomic sequence indicated by the sequence alignment dataset is upstream from a location, in the reference genomic sequence, indicated for another indel.

The process of FIG. 20 continues by creating a flattened aligned read based at least on removing from the read sequence any indels indicated by the initial alignment (2006), then determining one or more candidate realignments of the read sequence to the reference sequence (2008). Determining the candidate realignment(s) is done based on introducing, for each candidate realignment of the one or more candidate realignments, a respective at least one candidate indel of the one or more candidate indels into the flattened aligned read. The one or more candidate indels can include a plurality of candidate indels, and the determining the one or more candidate realignments can include commencing iteratively introducing the plurality of candidate indels into the flattened aligned read, where each iteration of the iteratively introducing provides a candidate realignment of the one or more candidate realignments by introducing into the flattened aligned read the respective at least one candidate indel for the candidate realignment. The iteratively introducing can introduce the plurality of indels in order of priority based on the prioritizing.

The iteratively introducing introduces permutations of one or more candidate indels of the plurality of candidate indels into the flattened read to obtain, for each permutation of the permutations, a different candidate realignment of the one or more candidate alignments.

The realignment processing (FIG. 20) eventually selects a best candidate realignment of the one or more candidate realignments (2010) based on selection criteria. Different criteria may be used for this selection than the criteria used in FIG. 19 to select between the initial alignment and the best candidate realignment. Thus, the selection of the best candidate realignment may be based on a first criteria of the one or more selection criteria, where the selected candidate realignment is the selected best candidate realignment, and wherein outputting (FIG. 19, 1912) selects between the initial alignment and the best realignment candidate based on a second criteria of the one or more selection criteria.

This selection of the best candidate realignment may include checking a provided candidate realignment to determine whether the aligned read of the provided candidate realignment, the aligned read having the introduced respective one or more candidate indels, aligns with the reference sequence with no bases mismatched as between the aligned read of the provided candidate realignment and the reference sequence. Based on determining that the aligned read of the provided candidate realignment aligns with the reference sequence with no bases mismatched, the iteratively introducing the candidate indel(s) into the flattened aligned read can halt, and the provided candidate realignment with no bases mismatched can be provides as the selected candidate realignment (2010). In these cases, the providing (FIG. 19, 1912) can output the selected candidate realignment based on the aligned read of the provided candidate realignment aligning with the reference sequence.

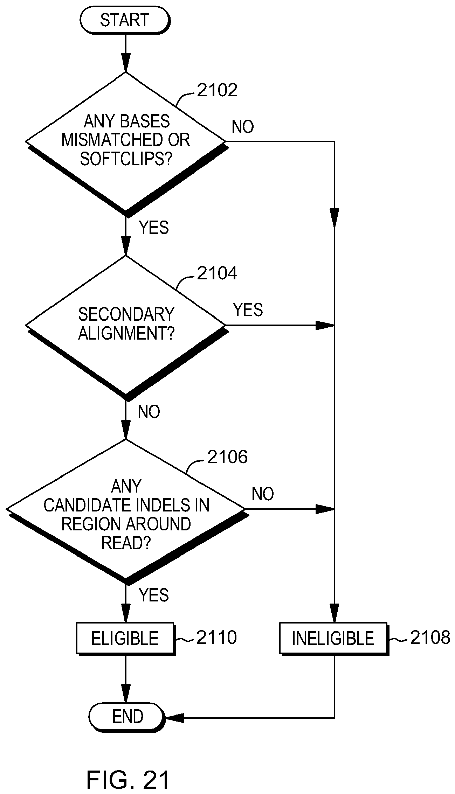

FIG. 21 depicts an example process for determining eligibility of an initial alignment to undergo realignment processing, in accordance with aspects described herein. This eligibility determination is performed at FIG. 19 (1906). The processing of FIG. 21 may be performed by one or more computer systems. The process begins by determining whether there are any (e.g. one or more) bases mismatched as between the aligned read of the initial alignment and the reference sequence or whether the aligned read includes a softclip (2102). If neither, then the process determines that the alignment is ineligible (2108) for realignment. Otherwise, there is/are mismatched base(s) and/or softclip(s), and the process continues by determining whether the alignment is a secondary alignment (2104). Whether or not the alignment is a secondary alignment may be indicated in the sequence alignment dataset, in one example. If it is identified that the alignment is a secondary alignment, the process determines that the alignment is ineligible (2108) for realignment. Otherwise, the process identifies that the initial alignment is not a secondary alignment, and continues to determine whether there are any candidate indel(s) around the aligned read in a region of bases of a reference genomic sequence of the sequence alignment dataset (2106). Thus, if there are none, the process determines that the alignment is ineligible (2108) for realignment. Otherwise, the process determines that the initial alignment is eligible (2110) for realignment processing and the process ends.

The example of FIG. 21 presents just some possible criteria for determining whether an alignment is eligible for realignment processing. The same or other criteria may be used singly or in combination with one or more other criteria.

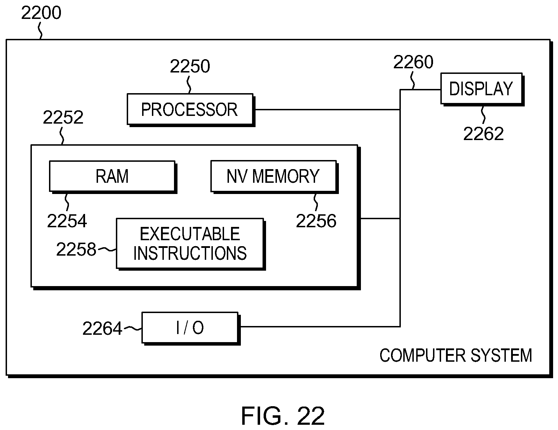

Processes described herein may be performed singly or collectively by one or more computer systems. FIG. 22 depicts one example of such a computer system and associated devices to incorporate and/or use aspects described herein. A computer system may also be referred to herein as a data processing device/system or computing device/system/node, or simply a computer. A computer system 2200 depicted in FIG. 22 may be implemented as one or more of a personal computer system, server computer system, thin client, thick client, hand-held or laptop device, mobile device, multiprocessor system, microprocessor-based system, set top box, programmable consumer electronic, network PC, minicomputer system, mainframe computer system, and/or distributed cloud computing environment that includes any of the above systems or devices, and the like.

The system 2200 includes one or more processors or processing units 2250 and a memory 2252 that includes volatile memory 2254 (e.g. Random Access Memory, RAM) and non-volatile memory 2056. Memory 2252 may further include removable/non-removable, volatile/non-volatile computer system storage media. Further, memory 2252 may include one or more readers for reading from and writing to a non-removable, non-volatile magnetic media, such as a hard drive, a magnetic disk drive for reading from and writing to a removable, non-volatile magnetic disk, and/or an optical disk drive for reading from or writing to a removable, non-volatile optical disk such as a CD-ROM, DVD-ROM. The system 2200 may also include a variety of computer readable tangible storage media. Such media may be any available media, such as volatile and non-volatile media, and removable and non-removable media.

Memory 2252 may include at least one program product having a set (e.g., at least one) of program modules implemented as executable instructions that, when executed, carry out functions described herein. Executable instructions 2258 may include an operating system, one or more application programs, other program modules, and program data or other types of software. Generally, program modules may include routines, programs, objects, components, logic, data structures, and so on, that perform particular tasks or implement particular abstract data types. Program modules may carry out functions, processes, methods and the like described herein including, but not limited to, sequencing data read realignment.

Components of the computer system 2200 may be coupled by an internal bus 2260 that may be implemented as one or more of any of several types of bus structures, including a memory bus or memory controller, a peripheral bus, an accelerated graphics port, and a processor or local bus using any of a variety of bus architectures.

Computer system 2200 may also communicate with one or more external devices such as a keyboard, a pointing device, a display 2262, etc.. and/or any devices (e.g., network card, modem, etc.) that enable computer system 2200 to communicate with one or more other computer systems, such as a server or other system hosted in a cloud computing environment. Such communication can occur via I/O interfaces 2264, which may include a network interfaces to interface to one or more networks such as a local area network (LAN), a general wide area network (WAN), and/or a public network (e.g., the Internet) via a suitable network adapter.

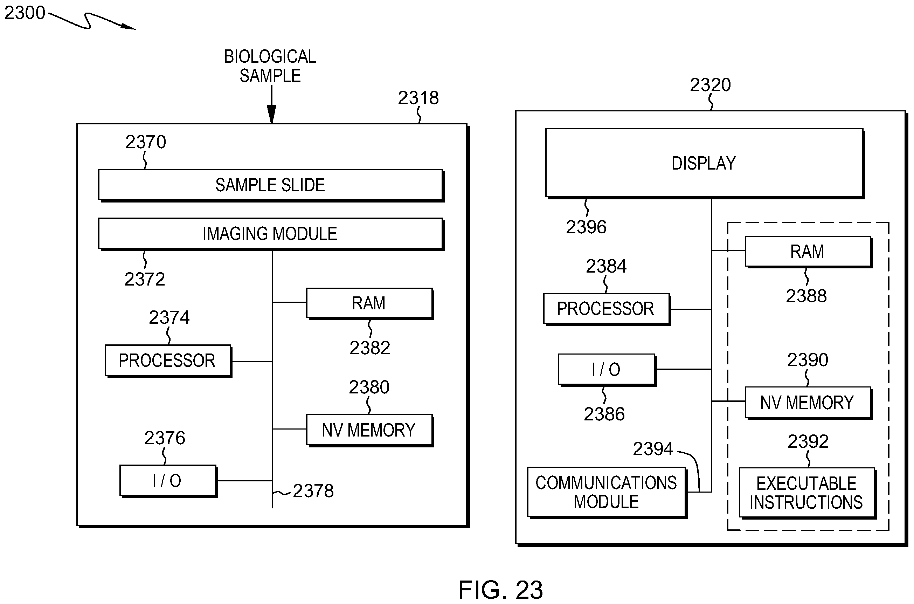

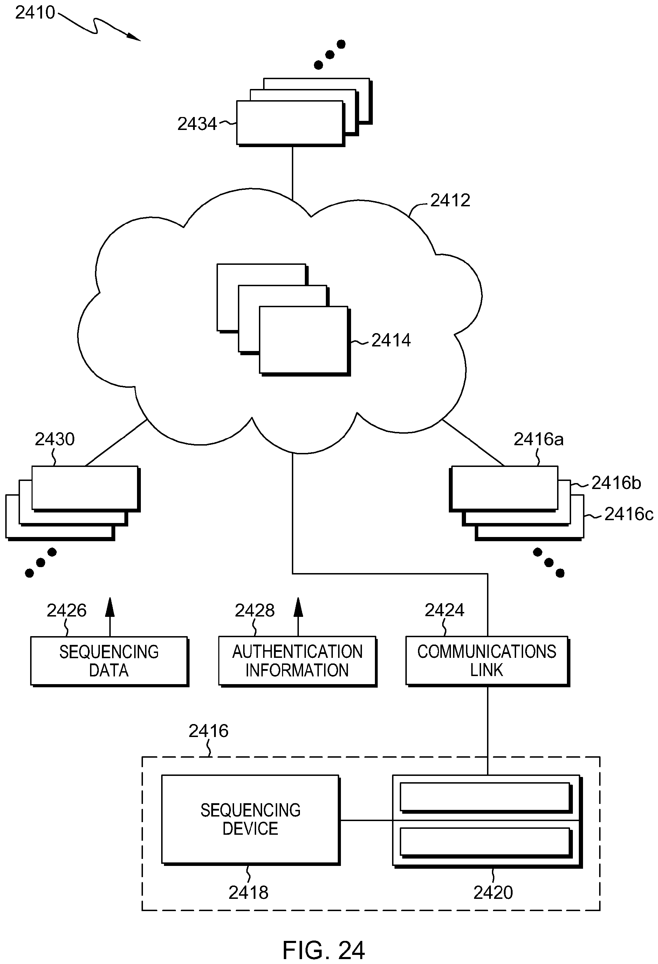

Further aspects of sequencing using a computer system are now described. FIG. 23 is a schematic diagram of the sequencing device 2300 that may be used in conjunction with, for instance, a cloud computing environment described with reference to FIG. 24. The sequence device 2300 may be implemented according to any sequencing technique, such as those incorporating sequencing-by-synthesis methods or sequencing by ligation techniques. Some embodiments can utilize nanopore sequencing, whereby target nucleic acid strands, or nucleotides exonucleolytically removed from target nucleic acids, pass through a nanopore. As the target nucleic acids or nucleotides pass through the nanopore, each type of base can be identified by measuring fluctuations in the electrical conductance of the pore. Yet other embodiments include detection of a proton released upon incorporation of a nucleotide into an extension product. For example, sequencing based on detection of released protons can use an electrical detector and associated techniques. Particular embodiments can utilize methods involving the real-time monitoring of DNA polymerase activity. Nucleotide incorporations can be detected through fluorescence resonance energy transfer (FRET) interactions between a fluorophore-bearing polymerase and y-phosphate-labeled nucleotides, or with zeromode waveguides. Other suitable alternative techniques include, for example, fluorescent in situ sequencing (FISSEQ), and Massively Parallel Signature Sequencing (MPSS). In particular embodiments, the sequencing device 16 may be a HiSeq, MiSeq, or HiScanSQ from Illumina Inc.

In the depicted embodiment, the sequencing device 2300 includes a separate sample processing device 2318 and an associated computer system 2320. However, as noted, these may be implemented as a single device. Further, the associated computer 2320 may be local to or networked with (e.g. as a cloud or other remoter offering) the sample processing device 2318. In some embodiments, the computer 2320 may be a cloud computing device that is remote from the sequencing device 2300. That is, the computer 2320 may be capable of communicating with the sequencing device 2300 through a cloud computing environment. In the depicted embodiment, the biological sample may be loaded into the sample processing device 2318 as a sample slide 2370 that is imaged to generate sequence data. For example, reagents that interact with the biological sample fluoresce at particular wavelengths in response to an excitation beam generated by an imaging module 2372 and thereby return radiation for imaging. For instance, the fluorescent components may be generated by fluorescently tagged nucleic acids that hybridize to complementary molecules of the components or to fluorescently tagged nucleotides that are incorporated into an oligonucleotide using a polymerase. As will be appreciated by those skilled in the art, the wavelength at which the dyes of the sample are excited and the wavelength at which they fluoresce will depend upon the absorption and emission spectra of the specific dyes. Such returned radiation may propagate back through directing optics. This retrobeam may generally be directed toward detection optics of the imaging module 2372.

The imaging module detection optics may be based upon any suitable technology, and may be, for example, a charged coupled device (CCD) sensor that generates pixilated image data based upon photons impacting locations in the device. However, it will be understood that any of a variety of other detectors may also be used including, but not limited to, a detector array configured for time delay integration (TDI) operation, a complementary metal oxide semiconductor (CMOS) detector, an avalanche photodiode (APD) detector, a Geiger-mode photon counter, or any other suitable detector. TDI mode detection can be coupled with line scanning. Other useful detectors are described, for example, in the references provided previously herein in the context of various nucleic acid sequencing methodologies.

The imaging module 2372 may be under processor control, e.g., via a processor 2374, and the sample receiving device 2318 may also include I/O controls 2376, an internal bus 2378, non-volatile memory 2380, RAM 2382 and any other memory structure such that the memory is capable of storing executable instructions, and other suitable hardware components that may be similar to those described with regard to FIG. 22. Further, the associated computer 2320 may also include a processor 2384, I/O controls 23386, a communications module 2387, and a memory architecture including RAM 2388 and non-volatile memory 2390, such that the memory architecture is capable of storing executable instructions 2392. The hardware components may be linked by an internal bus 2394, which may also link to the display 2396. In embodiments in which the sequencing device is implemented as an all-in-one device, certain redundant hardware elements may be eliminated.

Turning now to FIG. 24, a cloud computing environment 2410 for biological data is illustrated diagrammatically. As used herein, the term "cloud" or "cloud computing environment" may refer to various evolving arrangements, infrastructure, networks, and the like that will typically be based upon the Internet. The term may refer to any type of cloud, including client clouds, application clouds, platform clouds, infrastructure clouds, server clouds, and so forth. As will be appreciated by those skilled in the art, such arrangements will generally allow for use by owners or users of sequencing devices, provide software as a service (SaaS), provide various aspects of computing platforms as a service (PaaS), provide various network infrastructures as a service (IaaS) and so forth. Moreover, included in this term should be various types and business arrangements for these products and services, including public clouds, community clouds, hybrid clouds, and private clouds. Any or all of these may be serviced by third party entities. However, in certain embodiments, private clouds or hybrid clouds may allow for sharing of sequence data and services among authorized users.

A cloud facility 2412 includes a plurality of computer systems/nodes 2414. The computing resources of the nodes 2414 may be pooled to serve multiple consumers, with different physical and virtual resources dynamically assigned and reassigned according to consumer demand. Examples of resources include storage, processing, memory, network bandwidth, and virtual machines. The nodes 2414 may communicate with one another to distribute resources, and such communication and management of distribution of resources may be controlled by a cloud management module residing in one or more nodes 2414. The nodes 2414 may communicate via any suitable arrangement and protocol. Further, the nodes 2414 may include servers associated with one or more providers. For example, certain programs or software platforms may be accessed via a set of nodes 2414 provided by the owner of the programs while other nodes 2414 are provided by data storage companies. Certain nodes 2414 may also be overflow nodes that are used during higher load times.

In one embodiment, a cloud management module is responsible for load management and cloud resources. The load management may be implemented through consideration of a variety of factors, including user access level and/or total load in the cloud computing environment (peak times versus average load times). The project type may also be considered. In one embodiment, public health emergencies may be prioritized over other types of projects. Further, a user may manage costs by offering certain runs as lower priority that are held until cloud usage is below a certain threshold.

The cloud facility 2412 is configured to communicate with various users (e.g. user computer systems) for generating biological data. Such data may include sequence data generated via a sequencing device 2416, which in particular embodiments may include a sequencing device 2418 that includes a module to accept a biological sample and generate sequence data and an associated computer 2420 that includes executable instructions for analyzing or communicating the sequence data to the cloud facility 2412. It should be understood that, in certain embodiments, the sequencing device 2416 may also be implemented as an all-in-one device. The sequencing device 2416 is configured to communicate with the cloud facility 2412 via a suitable communications link 2424. The communication with the cloud facility 2412 may include communication via a local area network (LAN), a general wide area network (WAN), and/or a public network (e.g., the Internet) via the communications link 2424. In particular, the communications link 2424 sends sequence data 2426 and, in certain embodiments, authentication information 2428, to the cloud computing environment 2412. The authentication information may confirm that the sequencing device 2416 is a client of the cloud facility 2412.

As noted, the cloud facility 2412 may serve multiple users or clients with associated devices, e.g., devices 2416a, 2416b, and 2416c. Further, the cloud facility 2412 may also be accessed by other types of clients, such as secondary users 2430 or third party software holders. Accordingly, the cloud facility 2412 may provide different types of services depending on the access level of the particular client. A sequencing client may have access to storage and data analysis services, while a secondary user 2430 may have access only to shared or public sequences. Third party software holders may negotiate with sequencing clients to determine appropriate access privileges. For example, open source software may be offered for free or on limited license basis, while other types of software may be offered according to various fee or subscription bases.

Further, a primary user (or secondary user) may also interact with the cloud facility 2412 through any appropriate access device, such as a mobile device or other computer system that includes components similar to those described with regard to the computer 2420. That is, once the sequence data has been communicated to the cloud facility 2412, further interaction with and access to the sequence data may not necessarily be coupled to the sequence device 2416. Such embodiments may be beneficial in embodiments in which the owner of the biological sample and/or sequence data has contracted for sequencing, e.g., to a core laboratory facility. In such embodiments, the primary user may be the owner while the core laboratory facility associated with the sequencing device 2416 is at most a secondary user after the sequence data has been communicated to the cloud facility 2412. In certain embodiments, the sequence data may be accessed through security parameters such as a password-protected client account in the cloud facility 2412 or association with a particular institution or IP address. The sequence data may be accessed by downloading one or more files from the cloud facility 2412 or by logging into a web-based interface or software program that provides a graphical user display in which the sequence data is depicted as text, images, and/or hyperlinks. In such an embodiment, the sequence data may be provided to the primary or secondary user in the form of data packets transmitted via a communications link or network.

The cloud facility 2412 may execute user interaction software (e.g., via a web-based interface or application platform) that provides a graphical user interface for users and that facilitates access to sequence data, a community or group of researchers, data analysis programs, available third party software, and user selections for load balancing and instrument settings. For example, in particular embodiments, settings for a sequencing run on a sequencing device 2416 may be set via the cloud facility 2412. Accordingly, the cloud facility 2412 and an individual sequencing device 2416 may be capable of two-way communication. Such an embodiment may be particularly useful for controlling parameters of a remote sequencing run.

Results of a sequencing run and various analyses can be stored in files taking the form of FASTQ files, binary alignment files (bam), *.bcl, *.vcf, and/or *.csv files, as examples. The output files may be in formats that are compatible with sequence data viewing, modification, annotation, manipulation, alignment, and realignment software. Accordingly, the accessible sequence alignment dataset provided herein may be in the form of raw data, partially processed or processed data, and/or data files compatible with particular software programs. In this regard, a computer system, such as a computer system of or in communication with a sequencing device, or a cloud facility computer system, as examples, can obtain a bam or other sequencing alignment dataset and process the file by, for instance, reading its data and performing operations to carrying out aspects described herein. The computer system can then output a file having sequencing alignment data, for instance another bam file. Further, the output files may be compatible with other data sharing platforms or third party software.

Although various embodiments are described above, these are only examples. For example, computing environments of other architectures can be used to incorporate and use one or more embodiments.

The terminology used herein is for the purpose of describing particular embodiments only and is not intended to be limiting. As used herein, the singular forms "a", "an" and "the" are intended to include the plural forms as well, unless the context clearly indicates otherwise. It will be further understood that the terms "comprises" and/or "comprising", when used in this specification, specify the presence of stated features, integers, steps, operations, elements, and/or components, but do not preclude the presence or addition of one or more other features, integers, steps, operations, elements, components and/or groups thereof.

The corresponding structures, materials, acts, and equivalents of all means or step plus function elements in the claims below, if any, are intended to include any structure, material, or act for performing the function in combination with other claimed elements as specifically claimed. The description of one or more embodiments has been presented for purposes of illustration and description, but is not intended to be exhaustive or limited to in the form disclosed. Many modifications and variations will be apparent to those of ordinary skill in the art. The embodiment was chosen and described in order to best explain various aspects and the practical application, and to enable others of ordinary skill in the art to understand various embodiments with various modifications as are suited to the particular use contemplated.

## Claims

1. A computer-implemented method for sequencing data read realignment, the method comprising:
obtaining from a sequence alignment dataset an initial alignment of a read sequence to a reference sequence, the initial alignment comprising an aligned read;
performing realignment processing on the initial alignment, the realignment processing realigning the read sequence to the reference sequence to produce one or more candidate realignments that are different from the initial alignment, and the realignment processing comprising:
identifying one or more candidate indels, the one or more candidate indels comprising at least one indel in the aligned read and at least one indel aligned proximal to the aligned read as indicated by the sequence alignment dataset;
creating a flattened aligned read based at least on removing from the aligned read any indels indicated by the initial alignment; and
determining the one or more candidate realignments of the read sequence to the reference sequence based on introducing, for each candidate realignment of the one or more candidate realignments, a respective at least one candidate indel of the one or more candidate indels into the flattened aligned read;
and providing the initial alignment or a selected candidate realignment of the one or more candidate realignments based on one or more selection criteria,
wherein the one or more candidate indels comprise a plurality of candidate indels, and wherein the determining the one or more candidate realignments comprises commencing iteratively introducing the plurality of candidate indels into the flattened aligned read, wherein each iteration of the iteratively introducing provides a candidate realignment of the one or more candidate realignments by introducing into the flattened aligned read the respective at least one candidate indel for the candidate realignment.

2. The method of claim 1, wherein the iteratively introducing introduces permutations of one or more candidate indels of the plurality of candidate indels into the flattened aligned read to obtain, for each permutation of the permutations, a different candidate realignment of the one or more candidate alignments.

3. The method of claim 2, wherein the realignment processing further comprises:
checking a provided candidate realignment of the one or more candidate realignments to determine whether an aligned read of the provided candidate realignment, the aligned read of the provided candidate realignment having the introduced respective one or more candidate indels, aligns with the reference sequence with no bases mismatched as between the aligned read of the provided candidate realignment and the reference sequence;
halting the iteratively introducing based on determining that the aligned read of the provided candidate realignment aligns with the reference sequence with no bases mismatched; and
selecting the provided candidate realignment as the selected candidate realignment, wherein the providing outputs the selected candidate realignment based on the aligned read of the provided candidate realignment aligning with the reference sequence.

4. The method of claim 1, wherein the realignment processing further comprises prioritizing the plurality of indels for the iteratively introducing, wherein the iteratively introducing introduces the plurality of indels in order of priority based on the prioritizing.

5. The method of claim 4, wherein the prioritizing comprises one or more of:
prioritizing an indel indicated by a reference indel dataset to be a prior known indel over an indel not indicated by the reference indel dataset to be a prior known indel;
prioritizing an indel of longer length over an indel of shorter length;
prioritizing an indel indicated in a greater number of aligned reads of the sequence alignment dataset over an indel indicated in a lesser number of aligned reads of the sequence alignment dataset, or the prioritizing prioritizes an indel indicated in a greater proportion of aligned reads of the sequence alignment dataset that correspond to a location of the indel relative to the reference sequence over an indel indicated in a lesser proportion of aligned reads of the sequence alignment dataset; and
prioritizing, as between different indels indicated in a same number of aligned reads of the sequence alignment dataset, an indel whose location relative to a reference genomic sequence indicated by the sequence alignment dataset is upstream from a location, relative to the reference genomic sequence, indicated for another indel.

6. The method of claim 1, wherein the selection criteria is based at least in part on one or more of: number of bases mismatched, number of indels, location of indels relative to a reference genomic sequence indicated by the sequence alignment dataset, and number of softclipped bases.

7. The method of claim 1, 2, 3, 4, or 6, wherein the selection criteria prioritizes one or more of:
an alignment having no indels and only a single base mismatched over an alignment having one or more indels for the providing;
an alignment having a lesser number of bases mismatched over an alignment having a greater number of bases mismatched for the providing;
as between different alignments having a same number of bases mismatched, an alignment having a lesser number of softclips of a specified type over an alignment having a greater number of softclips of the specified type for the providing; and
as between different alignments having a same number of bases mismatched, an alignment having a lesser number of indels over an alignment having a greater number of indels for the providing.

8. The method of claim 1, 2, 3, 4, or 6, wherein the realignment processing further comprises selecting a best candidate realignment of the one or more candidate realignments based on a first criteria of the one or more selection criteria, wherein the selected candidate realignment is the selected best candidate realignment, and wherein the outputting selects between the initial alignment and the best realignment candidate based on a second criteria of the one or more selection criteria.

9. The method of claim 1, 2, 3, 4, or 6, further comprising determining whether the obtained initial alignment is eligible for realignment, the determining being based at least in part on one or more of:
identifying whether there are one or more bases mismatched as between the aligned read of the initial alignment and the reference sequence;
identifying whether the aligned read comprises a softclip;
identifying whether the initial alignment is not a secondary alignment; and
identifying whether there are candidate indels around the aligned read in a region of bases of a reference genomic sequence of the sequence alignment dataset.

10. The method of claim 1, 2, 3, 4, or 6, further comprising:
determining whether the obtained initial alignment is eligible for realignment, and performing the realignment processing and the providing the initial alignment or selected candidate realignment based on determining that the obtained initial alignment is eligible for realignment;
repeating, for each additional initial alignment of one or more additional initial alignments of the sequence alignment dataset, the obtaining and the determining whether the obtained additional initial alignment is eligible for realignment; and
performing processing for each additional initial alignment of the one or more additional initial alignments, the performing processing comprising (i) providing the additional initial alignment as is, absent performing the realignment processing, or (ii) performing the realignment processing and the providing the additional initial alignment or selected candidate realignment.

11. A computer system for sequencing data read realignment, the computer system comprising memory and at least one processor, the computer system configured to execute program instructions to perform a method comprising:
obtaining from a sequence alignment dataset an initial alignment of a read sequence to a reference sequence, the initial alignment comprising an aligned read;
performing realignment processing on the initial alignment, the realignment processing realigning the read sequence to the reference sequence to produce one or more candidate realignments that are different from the initial alignment, and the realignment processing comprising:
identifying one or more candidate indels, the one or more candidate indels comprising at least one indel in the aligned read and at least one indel aligned proximal to the aligned read as indicated by the sequence alignment dataset;
creating a flattened aligned read based at least on removing from the aligned read any indels indicated by the initial alignment; and
determining the one or more candidate realignments of the read sequence to the reference sequence based on introducing, for each candidate realignment of the one or more candidate realignments, a respective at least one candidate indel of the one or more candidate indels into the flattened aligned read;
and providing the initial alignment or a selected candidate realignment of the one or more candidate realignments based on one or more selection criteria,
wherein the one or more candidate indels comprise a plurality of candidate indels, and wherein the determining the one or more candidate realignments comprises commencing iteratively introducing the plurality of candidate indels into the flattened aligned read, wherein each iteration of the iteratively introducing provides a candidate realignment of the one or more candidate realignments by introducing into the flattened aligned read the respective at least one candidate indel for the candidate realignment.

12. The computer system of claim 11, wherein the iteratively introducing introduces permutations of one or more candidate indels of the plurality of candidate indels into the flattened aligned read to obtain, for each permutation of the permutations, a different candidate realignment of the one or more candidate alignments.

13. The computer system of claim 12, wherein the realignment processing further comprises:
checking a provided candidate realignment of the one or more candidate realignments to determine whether an aligned read of the provided candidate realignment, the aligned read of the provided candidate realignment having the introduced respective one or more candidate indels, aligns with the reference sequence with no bases mismatched as between the aligned read of the provided candidate realignment and the reference sequence;
halting the iteratively introducing based on determining that the aligned read of the provided candidate realignment aligns with the reference sequence with no bases mismatched; and
selecting the provided candidate realignment as the selected candidate realignment, wherein the providing outputs the selected candidate realignment based on the aligned read of the provided candidate realignment aligning with the reference sequence.

14. The computer system of claim 12 or 13, wherein the realignment processing further comprises prioritizing the plurality of indels for the iteratively introducing, wherein the iteratively introducing introduces the plurality of indels in order of priority based on the prioritizing.

## Patentansprüche

1. Computerimplementiertes Verfahren für Sequenzierdatenablesung-Realignment, das Verfahren umfassend:
Erlangen, von einem aus einem Sequenzalignment-Datensatz eines anfänglichen Alignments einer Ablesesequenz zu einer Referenzsequenz, das anfängliche Alignment umfassend eine alignierte Ablesung;
Ausführen einer Realignment-Verarbeitung an dem anfänglichen Alignment,
wobei die Realignment-Verarbeitung die Ablesesequenz an der Referenzsequenz realigniert, um eine oder mehrere Kandidaten-Realignments zu erzeugen, die sich von der anfänglichen Alignment unterscheiden, und die Realignment-Verarbeitung umfassend:
Identifizieren eines oder mehrerer Kandidaten-Indels, wobei das eine oder die mehreren Kandidaten-Indels mindestens ein Indel in der alignierten Ablesung und mindestens ein Indel, das proximal zu der alignierten Ablesung aligniert ist, umfassen, wie es durch den Sequenzalignment-Datensatz angegeben ist;
Erzeugen einer abgeflachten alignierten Ablesung zumindest basierend auf einem Entfernen von Indels aus der alignierten Ablesung, die durch das anfängliche Alignment angegeben werden; und
Bestimmen des einen oder der mehreren Kandidaten-Realignments der Ablesesequenz an der Referenzsequenz basierend auf einem Einführen, für jedes Kandidaten-Realignment der einen oder der mehreren Kandidaten-Realignments, eines jeweiligen mindestens einen Kandidaten-Indels des einen oder der mehreren Kandidaten-Indels in die abgeflachte alignierte Ablesung;
und Bereitstellen des anfänglichen Alignments oder eines ausgewählten Kandidaten-Realignments des einen oder der mehreren Kandidaten-Realignments basierend auf einem oder mehreren Auswahlkriterien, wobei das eine oder die mehreren Kandidaten-Indels eine Vielzahl von Kandidaten-Indels umfassen und wobei das Bestimmen des einen oder der mehreren Kandidaten-Realignments ein iteratives Einführen der Vielzahl von Kandidaten-Indels in die abgeflachte alignierte Ablesung umfasst, wobei jede Iteration des iterativen Einführens ein Kandidaten-Realignment des einen oder der mehreren Kandidaten-Realignments durch Einführen des jeweiligen mindestens einen Kandidaten-Indels für das Kandidaten-Realignment in die abgeflachte alignierte Ablesung bereitstellt.

2. Verfahren nach Anspruch 1, wobei das iterative Einführen Permutationen von einer oder mehreren Kandidaten-Indels der Vielzahl von Kandidaten-Indels in die abgeflachte alignierte Ablesung einführt, um für jede Permutation der Permutationen ein unterschiedliches Kandidaten-Realignment des einen oder der mehreren Kandidaten-Alignments zu erlangen.

3. Verfahren nach Anspruch 2, wobei die Realignment-Verarbeitung ferner Folgendes umfasst:
Überprüfen eines bereitgestellten Kandidaten-Realignments des einen oder der mehreren Kandidaten-Realignments, um zu bestimmen, ob eine alignierte Ablesung des bereitgestellten Kandidaten-Realignments, die alignierte Ablesung des bereitgestellten Kandidaten-Realignments, das die eingeführte jeweilige eine oder mehreren Kandidaten-Indels aufweist, mit der Referenzsequenz ohne Basenfehlpaarung aligniert ist, wie zwischen der alignierten Ablesung des bereitgestellten Kandidaten-Realignments und der Referenzsequenz;
Anhalten des iterativen Einführens basierend auf einem Bestimmen, dass die alignierte Ablesung des bereitgestellten Kandidaten-Realignment ohne Basenfehlpaarung mit der Referenzsequenz aligniert ist; und
Auswählen des bereitgestellten Kandidaten-Realignments als das ausgewählte Kandidaten-Realignment, wobei das Bereitstellen das ausgewählte Kandidaten-Realignment basierend auf einer alignierten Ablesung des bereitgestellten Kandidaten-Realignments, das mit der Referenzsequenz aligniert ist, ausgibt.

4. Verfahren nach Anspruch 1, wobei die Realignment-Verarbeitung ferner ein Priorisieren der Vielzahl von Indels für das iterative Einführen umfasst, wobei das iterative Einführen die Vielzahl von Indels in der Reihenfolge der Priorität basierend auf dem Priorisieren einführt.

5. Verfahren nach Anspruch 4, wobei das Priorisieren eines oder mehrere von Folgenden umfasst:
Priorisieren eines Indels, das durch einen Referenzindel-Datensatz angegeben wird, um ein vorher bekanntes Indel zu sein, gegenüber einem Indel, das nicht durch den Referenzindel-Datensatz angegeben wird, um ein vorher bekanntes Indel zu sein;
Priorisieren eines Indels mit längerer Länge gegenüber einem Indel mit kürzerer Länge;
Priorisieren eines Indels, das in einer größeren Anzahl alignierter Lesevorgänge des Sequenzalignment-Datensatzes angegeben ist, gegenüber einem Indel, das in einer geringeren Anzahl alignierter Lesevorgänge des Sequenzalignment-Datensatzes angegeben ist, oder Priorisieren eines Indels, das in einem größeren Anteil alignierter Lesevorgänge des Sequenzalignment-Datensatzes angegeben ist, die einer Position des Indels in Bezug auf die Referenzsequenz entspricht, gegenüber einem Indel, das in einem geringeren Anteil alignierter Lesevorgänge des Sequenzalignment-Datensatzes angegeben ist; und
Priorisieren, wie zwischen verschiedenen Indeln, die in einer gleichen Anzahl von alignierten Ablesungen des Sequenzalignment-Datensatzes angegeben sind, eines Indels, dessen Position in Bezug auf eine Referenzgenomsequenz, die durch den Sequenzalignment-Datensatz angegeben ist, stromaufwärts von einer Position in Bezug auf die Referenzgenomsequenz liegt, die für ein anderes Indel angegeben ist.

6. Verfahren nach Anspruch 1, wobei das Auswahlkriterium zumindest teilweise auf einem oder mehreren von Folgendem basiert: Anzahl fehlgepaarter Basen, Anzahl an Indels, Position der Indels in Bezug auf eine genomische Referenzsequenz, die durch den Sequenzalignment-Datensatz angegeben wird, und Anzahl an Softclipped-Basen.

7. Verfahren nach Anspruch 1, 2, 3, 4 oder 6, wobei die Auswahlkriterien eines oder mehrere von Folgenden priorisieren:
ein Alignment, das keine Indels und nur eine einzige fehlgepaarte Base aufweist, gegenüber einem Alignment, das ein oder mehrere Indels zum Bereitstellen aufweist;
ein Alignment, das eine geringere Anzahl von fehlgepaarten Base aufweist, gegenüber einem Alignment, das eine größere Anzahl von fehlgepaarten Basen zum Bereitstellen bereitstellt;
wie zwischen verschiedenen Alignments, die eine gleiche Anzahl von fehlgepaarten Basen aufweisen, wobei ein Alignment eine geringere Anzahl von Softclips eines bestimmten Typs aufweist, gegenüber einem Alignment, das eine größere Anzahl von Softclips des bestimmten Typs zum Bereitstellen aufweist; und
wie zwischen verschiedenen Alignments, die eine gleiche Anzahl von fehlgepaarten Basen aufweisen, wobei ein Alignment eine geringere Anzahl von Indels gegenüber einem Alignment aufweist, das eine größere Anzahl von Indels zum Bereitstellen aufweist.

8. Verfahren nach Anspruch 1, 2, 3, 4 oder 6, wobei die Realignment-Verarbeitung ferner ein Auswählen eines besten Kandidaten-Realignment des einen oder der mehreren Kandidaten-Realignments basierend auf einem ersten Kriterium des einen oder der mehreren Auswahlkriterien umfasst, wobei das ausgewählte Kandidaten-Realignment das ausgewählte beste Kandidaten-Realignment ist, und wobei das Ausgeben zwischen dem anfänglichen Alignment und dem besten Realignment-Kandidaten basierend auf einem zweiten Kriterium des einen oder der mehreren Auswahlkriterien auswählt.

9. Verfahren nach Anspruch 1, 2, 3, 4 oder 6, ferner umfassend ein Bestimmen, ob das erlangte anfängliche Alignment für ein Realignment geeignet ist, wobei das Bestimmen zumindest teilweise auf einem oder mehreren von Folgendem basiert:
Identifizieren, ob es eine oder mehrere Basen gibt, die zwischen der alignierten Ablesung des anfänglichen Alignments und der Referenzsequenz fehlgepaart sind;
Identifizieren, ob die alignierte Ablesung einen Softclip umfasst;
Identifizieren, ob das anfängliche Alignment kein sekundäres Alignment ist; und
Identifizieren, ob Kandidaten-Indels um die alignierte Ablesung in einer Region von Basen einer genomischen Referenzsequenz des Sequenzalignment-Datensatzes vorhanden sind.

10. Verfahren nach Anspruch 1, 2, 3, 4 oder 6, ferner umfassend:
Bestimmen, ob das erlangte anfängliche Alignment für ein Realignment in Frage kommt, und Ausführen der Realignment-Verarbeitung und das Bereitstellen des anfänglichen Alignments oder des ausgewählten Kandidaten-Realignments basierend auf einem Bestimmen, dass das erlangte anfängliche Alignment für ein Realignment in Frage kommt;
Wiederholen, für jedes zusätzliches anfängliches Alignment von einer oder mehreren zusätzlichen anfänglichen Alignments des Sequenzalignment-Datensatzes, des Erlangens und Bestimmens, ob das erlangte zusätzliche anfängliche Alignment für ein Realignment in Frage kommt; und
Ausführen eines Verarbeitens für jedes zusätzliches anfängliches Alignment des einen oder der mehreren zusätzlichen anfänglichen Alignments, wobei das Ausführen der Verarbeitung umfassend (i) Bereitstellen des zusätzlichen anfänglichen Alignments, wie es ist, bei Fehlen Ausführen der Realignment-Verarbeitung oder (ii) Ausführen der Realignment-Verarbeitung und Bereitstellen des zusätzlichen anfänglichen Alignments oder ausgewählten Kandidaten-Realignments umfasst.

11. Computersystem für Sequenzierdatenablesung-Realignment, das Computersystem umfassend einen Speicher und mindestens einen Prozessor, wobei das Computersystem konfiguriert ist, um Programmanweisungen auszuführen, um ein Verfahren durchzuführen, umfassend:
Erlangen, von einem aus einem Sequenzalignment-Datensatz eines anfänglichen Alignments einer Ablesesequenz zu einer Referenzsequenz, das anfängliche Alignment umfassend eine alignierte Ablesung;
Ausführen einer Realignment-Verarbeitung an dem anfänglichen Alignment, wobei die Realignment-Verarbeitung die Ablesesequenz an der Referenzsequenz realigniert, um eine oder mehrere Kandidaten-Realignments zu erzeugen, die sich von der anfänglichen Alignment unterscheiden, und die Realignment-Verarbeitung umfassend:
Identifizieren eines oder mehrerer Kandidaten-Indels, wobei das eine oder die mehreren Kandidaten-Indels mindestens ein Indel in der alignierten Ablesung und mindestens ein Indel, das proximal zu der alignierten Ablesung aligniert ist, umfassen, wie es durch den Sequenzalignment-Datensatz angegeben ist;
Erzeugen einer abgeflachten alignierten Ablesung zumindest basierend auf einem Entfernen von Indels aus der alignierten Ablesung, die durch das anfängliche Alignment angegeben werden; und
Bestimmen des einen oder der mehreren Kandidaten-Realignments der Ablesesequenz an der Referenzsequenz basierend auf einem Einführen, für jedes Kandidaten-Realignment der einen oder der mehreren Kandidaten-Realignments, eines jeweiligen mindestens einen Kandidaten-Indels des einen oder der mehreren Kandidaten-Indels in die abgeflachte alignierte Ablesung;
und Bereitstellen des anfänglichen Alignments oder eines ausgewählten Kandidaten-Realignments des einen oder der mehreren Kandidaten-Realignments basierend auf einem oder mehreren Auswahlkriterien, wobei das eine oder die mehreren Kandidaten-Indels eine Vielzahl von Kandidaten-Indels umfassen und wobei das Bestimmen des einen oder der mehreren Kandidaten-Realignments ein iteratives Einführen der Vielzahl von Kandidaten-Indels in die abgeflachte alignierte Ablesung umfasst, wobei jede Iteration des iterativen Einführens ein Kandidaten-Realignment des einen oder der mehreren Kandidaten-Realignments durch Einführen des jeweiligen mindestens einen Kandidaten-Indels für das Kandidaten-Realignment in die abgeflachte alignierte Ablesung bereitstellt.

12. Computersystem nach Anspruch 11, wobei das iterative Einführen Permutationen von einer oder mehreren Kandidaten-Indels der Vielzahl von Kandidaten-Indels in die abgeflachte alignierte Ablesung einführt, um für jede Permutation der Permutationen ein unterschiedliches Kandidaten-Realignment des einen oder der mehreren Kandidaten-Alignments zu erlangen.

13. Computersystem nach Anspruch 12, wobei die Realignment-Verarbeitung ferner Folgendes umfasst:
Überprüfen eines bereitgestellten Kandidaten-Realignments des einen oder der mehreren Kandidaten-Realignments, um zu bestimmen, ob eine alignierte Ablesung des bereitgestellten Kandidaten-Realignments, die alignierte Ablesung des bereitgestellten Kandidaten-Realignments, das die eingeführte jeweilige eine oder mehreren Kandidaten-Indels aufweist, mit der Referenzsequenz ohne Basenfehlpaarung aligniert ist, wie zwischen der alignierten Ablesung des bereitgestellten Kandidaten-Realignments und der Referenzsequenz;
Anhalten des iterativen Einführens basierend auf einem Bestimmen, dass die alignierte Ablesung des bereitgestellten Kandidaten-Realignment ohne Basenfehlpaarung mit der Referenzsequenz aligniert ist; und
Auswählen des bereitgestellten Kandidaten-Realignments als das ausgewählte Kandidaten-Realignment, wobei das Bereitstellen das ausgewählte Kandidaten-Realignment basierend auf einer alignierten Ablesung des bereitgestellten Kandidaten-Realignments, das mit der Referenzsequenz aligniert ist, ausgibt.

14. Computersystem nach Anspruch 12 oder 13, wobei die Realignment-Verarbeitung ferner ein Priorisieren der Vielzahl von Indels für das iterative Einführen umfasst, wobei das iterative Einführen die Vielzahl von Indels in der Reihenfolge der Priorität basierend auf einem Priorisieren einführt.

## Revendications

1. Procédé mis en oeuvre par ordinateur pour séquencer un réalignement de lecture de données, le procédé comprenant les étapes consistant à :
obtenir à partir d'un ensemble de données d'alignement de séquences un alignement initial d'une séquence de lecture à une séquence de référence, l'alignement initial comprenant une lecture alignée ;
effectuer un traitement de réalignement sur l'alignement initial, le traitement de réalignement réalignant la séquence de lecture à la séquence de référence pour produire un ou plusieurs réalignements candidats qui sont différents de l'alignement initial, et le traitement de réalignement comprenant :
identifier un ou plusieurs indels candidats, l'un ou plusieurs indels candidats comprenant au moins un indel dans la lecture alignée et au moins un indel aligné à proximité de la lecture alignée comme indiqué par l'ensemble de données d'alignement de séquences ;
créer une lecture alignée aplatie sur la base au moins de l'élimination à partir de la lecture alignée de tous les indels indiqués par l'alignement initial de la lecture alignée ; et
déterminer l'un ou plusieurs réalignements candidats de la séquence de lecture à la séquence de référence sur la base de l'introduction, pour chaque réalignement candidat de l'un ou plusieurs réalignements candidats, d'au moins un indel candidat respectif de l'un ou plusieurs indels candidats dans la lecture alignée aplatie ;
et fournir l'alignement initial ou un réalignement candidat sélectionné de l'un ou plusieurs réalignements candidats sur la base d'un ou de plusieurs critères de sélection,
dans lequel l'un ou plusieurs indels candidats comprennent une pluralité d'indels candidats, et dans lequel la détermination de l'un ou plusieurs réalignements candidats comprend le début de l'introduction itérative de la pluralité d'indels candidats dans la lecture alignée aplatie, dans lequel chaque itération de l'introduction itérative fournit un réalignement candidat de l'un ou plusieurs réalignements candidats en introduisant dans la lecture alignée aplatie l'au moins un indel candidat respectif pour le réalignement candidat.

2. Procédé selon la revendication 1, dans lequel l'introduction itérative introduit des permutations d'un ou plusieurs indels candidats de la pluralité d'indels candidats dans la lecture alignée aplatie pour obtenir, pour chaque permutation des permutations, un réalignement candidat différent de l'un ou plusieurs alignements candidats.

3. Appareil selon la revendication 2, dans lequel le traitement de réalignement comprend en outre les étapes consistant à :
vérifier un réalignement candidat fourni de l'un ou plusieurs réalignements candidats pour déterminer si une lecture alignée du réalignement candidat fourni, la lecture alignée du réalignement candidat fourni ayant l'un ou plusieurs indels candidats respectifs introduits, s'aligne avec la séquence de référence sans bases non appariées entre la lecture alignée du réalignement candidat fourni et la séquence de référence ;
arrêter l'introduction itérative sur la base de la détermination que la lecture alignée du réalignement candidat fourni s'aligne avec la séquence de référence sans bases non appariées ; et
sélectionner le réalignement candidat fourni comme réalignement candidat sélectionné, dans lequel la fourniture produit en sortie le réalignement candidat sélectionné sur la base de la lecture alignée du réalignement candidat fourni s'alignant avec la séquence de référence.

4. Procédé selon la revendication 1, dans lequel le traitement de réalignement comprend en outre la priorisation de la pluralité d'indels pour l'introduction itérative, dans lequel l'introduction itérative introduit la pluralité d'indels par ordre de priorité sur la base de la priorisation.

5. Procédé selon la revendication 4, dans lequel la priorisation comprend l'un ou plusieurs des étapes suivantes :
donner la priorité à un indel indiqué par un ensemble de données d'indel de référence comme étant un indel connu antérieurement au lieu d'un indel non indiqué par l'ensemble de données d'indel de référence comme étant un indel connu antérieurement ;
donner la priorité à un indel de plus grande longueur au lieu d'un indel de plus petite longueur ;
donner la priorité à un indel indiqué dans un plus grand nombre de lectures alignées de l'ensemble de données d'alignement de séquences au lieu d'un indel indiqué dans un plus petit nombre de lectures alignées de l'ensemble de données d'alignement de séquences, ou donner la priorité à la priorisation d'un indel indiqué dans une plus grande proportion de lectures alignées de l'ensemble de données d'alignement de séquences qui correspondent à un emplacement de l'indel par rapport à la séquence de référence au lieu d'un indel indiqué dans une plus petite proportion de lectures alignées de l'ensemble de données d'alignement de séquences ; et prioriser, parmi différents indels indiqués dans un même nombre de lectures alignées de l'ensemble de données d'alignement de séquences, un indel dont l'emplacement par rapport à une séquence génomique de référence indiquée par l'ensemble de données d'alignement de séquences est en amont d'un emplacement, par rapport à la séquence génomique de référence, indiqué pour un autre indel.

6. Procédé selon la revendication 1, dans lequel les critères de sélection sont basés au moins en partie sur un ou plusieurs éléments parmi : le nombre de bases non appariées, le nombre d'indels, l'emplacement des indels par rapport à une séquence génomique de référence indiquée par l'ensemble de données d'alignement de séquences, et le nombre de bases écrêtées.

7. Procédé selon la revendication 1, 2, 3, 4 ou 6, dans lequel les critères de sélection donnent la priorité à un ou plusieurs des éléments suivants :
un alignement n'ayant pas d'indels et seulement une seule base non appariée au lieu d'un alignement ayant un ou plusieurs indels pour la fourniture ;
un alignement ayant un plus petit nombre de bases non appariées au lieu d'un alignement ayant un plus grand nombre de bases non appariées pour la fourniture ;
parmi différents alignements ayant un même nombre de bases non appariées, un alignement ayant un plus petit nombre d'écrêtages d'un type spécifié au lieu d'un alignement ayant un plus grand nombre d'écrêtages du type spécifié pour la fourniture ; et
parmi différents alignements ayant un même nombre de bases non appariées, un alignement ayant un plus petit nombre d'indels au lieu d'un alignement ayant un plus grand nombre d'indels pour la fourniture.

8. Procédé selon la revendication 1, 2, 3, 4 ou 6, dans lequel le traitement de réalignement comprend en outre les étapes consistant à sélectionner un meilleur réalignement candidat de l'un ou plusieurs réalignements candidats sur la base d'un premier critère de l'un ou plusieurs critères de sélection, dans lequel le réalignement candidat sélectionné est le meilleur réalignement candidat sélectionné, et dans lequel la production en sortie sélectionne entre l'alignement initial et le meilleur candidat de réalignement sur la base d'un second critère de l'un ou plusieurs critères de sélection.

9. Procédé selon la revendication 1, 2, 3, 4 ou 6, comprenant en outre les étapes consistant à déterminer si l'alignement initial obtenu est admissible à un réalignement, la détermination étant basée au moins en partie sur une ou plusieurs des étapes suivantes :
identifier s'il existe une ou plusieurs bases non appariées entre la lecture alignée de l'alignement initial et la séquence de référence ;
identifier si la lecture alignée comprend un écrêtage ;
déterminer si l'alignement initial n'est pas un alignement secondaire ; et identifier s'il y a des indels candidats autour de la lecture alignée dans une région de bases d'une séquence génomique de référence de l'ensemble de données d'alignement de séquences.

10. Procédé selon la revendication 1, 2, 3, 4 ou 6, comprenant en outre les étapes consistant à :
déterminer si l'alignement initial obtenu est admissible à un réalignement, et effectuer le traitement de réalignement et la fourniture de l'alignement initial ou du réalignement candidat sélectionné sur la base de la détermination que l'alignement initial obtenu est admissible à un réalignement ;
répéter, pour chaque alignement initial supplémentaire d'un ou plusieurs alignements initiaux supplémentaires de l'ensemble de données d'alignement de séquences, l'obtention et la détermination de l'admissibilité à un réalignement de l'alignement initial supplémentaire obtenu ; et
effectuer un traitement pour chaque alignement initial supplémentaire de l'un ou plusieurs alignements initiaux supplémentaires, l'exécution du traitement comprenant les étapes de (i) fourniture de l'alignement initial supplémentaire tel quel, en l'absence d'exécution du traitement de réalignement, ou (ii) d'exécution du traitement de réalignement et de la fourniture de l'alignement initial supplémentaire ou du réalignement candidat sélectionné.

11. Système informatique pour séquencer un réalignement de lecture de données, le système informatique comprenant une mémoire et au moins un processeur, le système informatique étant configuré pour exécuter des instructions de programme afin de mettre en oeuvre un procédé comprenant les étapes consistant à :
obtenir à partir d'un ensemble de données d'alignement de séquences un alignement initial d'une séquence de lecture à une séquence de référence, l'alignement initial comprenant une lecture alignée ;
effectuer un traitement de réalignement sur l'alignement initial, le traitement de réalignement réalignant la séquence de lecture à la séquence de référence pour produire un ou plusieurs réalignements candidats qui sont différents de l'alignement initial, et le traitement de réalignement comprenant :
identifier un ou plusieurs indels candidats, l'un ou plusieurs indels candidats comprenant au moins un indel dans la lecture alignée et au moins un indel aligné à proximité de la lecture alignée comme indiqué par l'ensemble de données d'alignement de séquences ;
créer une lecture alignée aplatie sur la base au moins de l'élimination à partir de la lecture alignée de tous les indels indiqués par l'alignement initial de la lecture alignée ; et
déterminer l'un ou plusieurs réalignements candidats de la séquence de lecture à la séquence de référence sur la base de l'introduction, pour chaque réalignement candidat de l'un ou plusieurs réalignements candidats, d'au moins un indel candidat respectif de l'un ou plusieurs indels candidats dans la lecture alignée aplatie ;
et fournir l'alignement initial ou un réalignement candidat sélectionné de l'un ou plusieurs réalignements candidats sur la base d'un ou de plusieurs critères de sélection,
dans lequel l'un ou plusieurs indels candidats comprennent une pluralité d'indels candidats, et dans lequel la détermination de l'un ou plusieurs réalignements candidats comprend le début de l'introduction itérative de la pluralité d'indels candidats dans la lecture alignée aplatie, dans lequel chaque itération de l'introduction itérative fournit un réalignement candidat de l'un ou plusieurs réalignements candidats en introduisant dans la lecture alignée aplatie l'au moins un indel candidat respectif pour le réalignement candidat.

12. Système informatique selon la revendication 11, dans lequel l'introduction itérative introduit des permutations d'un ou plusieurs indels candidats de la pluralité d'indels candidats dans la lecture alignée aplatie pour obtenir, pour chaque permutation des permutations, un réalignement candidat différent de l'un ou plusieurs alignements candidats.

13. Système informatique selon la revendication 12, dans lequel le traitement de réalignement comprend en outre les étapes consistant à :
vérifier un réalignement candidat fourni de l'un ou plusieurs réalignements candidats pour déterminer si une lecture alignée du réalignement candidat fourni, la lecture alignée du réalignement candidat fourni ayant l'un ou plusieurs indels candidats respectifs introduits, s'aligne avec la séquence de référence sans bases non appariées entre la lecture alignée du réalignement candidat fourni et la séquence de référence ;
arrêter l'introduction itérative sur la base de la détermination que la lecture alignée du réalignement candidat fourni s'aligne avec la séquence de référence sans bases non appariées ; et
sélectionner le réalignement candidat fourni comme réalignement candidat sélectionné, dans lequel la fourniture produit en sortie le réalignement candidat sélectionné sur la base de la lecture alignée du réalignement candidat fourni s'alignant avec la séquence de référence.

14. Système informatique selon la revendication 12 ou 13, dans lequel le traitement de réalignement comprend en outre la priorisation de la pluralité d'indels pour l'introduction itérative, dans lequel l'introduction itérative introduit la pluralité d'indels par ordre de priorité sur la base de la priorisation.
